(19) **Europäisches Patentamt**

**European Patent Office**

**Office européen des brevets**

(11) **EP 1 289 515 B1**

(12) **FASCICULE DE BREVET EUROPEEN**

(45) Date de publication et mention
de la délivrance du brevet:
**13.10.2004 Bulletin 2004/42**

(51) Int Cl.⁷: **A61K 31/19**, A61K 47/36,
C08B 37/02, A61K 47/48,
A61P 35/00

(21) Numéro de dépôt: **01940634.7**

(22) Date de dépôt: **30.05.2001**

(86) Numéro de dépôt international:
**PCT/FR2001/001672**

(87) Numéro de publication internationale:
**WO 2001/091742 (06.12.2001 Gazette 2001/49)**

(54) **COMPOSITION PHARMACEUTIQUE CONTENANT AU MOINS UN POLYMERE ASSOCIE OU CONJUGUE A AU MOINS UN SEL D'UN ACIDE PHENYLALKYLCARBOXYLIQUE, POLYMERES CONJUGUES ET LEURS APPLICATIONS**

PHARMAZEUTISCHE ZUSAMMENSETZUNG DIE EIN POLYMER ASSOZIERT ODER KONJUGIERT AN EINEM SALZ EINER PHENYLALKYLCARBOXYLSÄURE ENTHÄLT; DIE KONJUGIERTE POLYMERE UND IHRE ANWENDUNGEN

PHARMACEUTICAL COMPOSITION CONTAINING AT LEAST A POLYMER ASSOCIATED OR CONJUGATED WITH AT LEAST A PHENYLALKYLCARBOXYLIC ACID SALT, CONJUGATE POLYMERS AND USES THEREOF

(84) Etats contractants désignés:
**AT BE CH CY DE DK ES FI FR GB GR IE IT LI LU MC NL PT SE TR**

(30) Priorité: **02.06.2000 FR 0007117**

(43) Date de publication de la demande:
**12.03.2003 Bulletin 2003/11**

(73) Titulaire: **BIODEX**
**92300 Levallois Perret (FR)**

(72) Inventeurs:
• **AVRAMOGLOU, Thierry**
**F-95410 Groslay (FR)**
• **BAGHERI, Rozita**
**Houston, TX 77025 (US)**
• **CHAUBET, Frédéric**
**F-95600 Eaubonne (FR)**
• **CREPIN, Michel**
**F-75013 Paris (FR)**
• **DAHRI-CORREIA, Latifa**
**F-59230 St Amand les Eaux (FR)**
• **DIBENEDETTO, Mélanie**
**F-93220 Gagny (FR)**
• **GERVELAS, Claudia**
**F-75019 Paris (FR)**
• **HUYNH, Rémi**
**F-59158 Mortagne du Nord (FR)**
• **JOZEFONVICZ, Jacqueline**
**F-60260 Lamorlaye (FR)**

(74) Mandataire: **Goulard, Sophie et al**
**Cabinet ORES,**
**36,rue de St Pétersbourg**
**75008 Paris Cedex (FR)**

(56) Documents cités:
EP-A- 0 331 471          EP-A- 0 485 158
WO-A-91/12011            WO-A-95/00177
CA-A- 2 091 410          US-A- 3 793 065

• MORA M ET AL: "POLYMERIC PRODRUGS. 8A) SYNTHESIS AND HYDROLYTIC BEHAVIOUR OF DEXTRAN-BOUND ANTICANCER AGENTS" MAKROMOLEKULARE CHEMIE, MACROMOLECULAR CHEMISTRY AND PHYSICS,HUTHIG UND WEPF VERLAG, BASEL,CH, vol. 191, no. 5, mai 1990 (1990-05), pages 1051-1056, XP000159595 ISSN: 0025-116X
• ASHINI GADRE ET AL.: "Binding of Substituted Acetic Acids to alpha-Cyclodextrin in aqueous solution" JOURNAL OF PHARMACEUTICAL SCIENCES, vol. 86, no. 11, 1 novembre 1997 (1997-11-01), pages 1210-1214, XP001024106

**Description**

La présente invention est relative à une composition pharmaceutique contenant au moins un polymère associé ou conjugué à au moins un dérivé d'un acide phénylalkylcarboxylique, à de nouveaux polymères conjugués à au moins un dérivé d'un acide phénylalkylcarboxylique et à leurs applications notamment dans le traitement du cancer.

On entend par polymère au sens de la présente invention, des macromolécules comportant au moins une fonction hydroxyle.

Les polymères comportant au moins une fonction hydroxyle, c'est le cas en particulier des polyalcools et des polysaccharides, peuvent se présenter sous leur forme native ou bien être fonctionnalisés, étant donné que la ou les fonctions hydroxyles qu'ils comportent sont aisément substituables par estérification ou éthérification.

Les polysaccharides sont répartis en deux classes suivant qu'ils se composent de molécules d'oses toutes identiques (homopolyosides), ou différentes (hétéropolyosides).

Parmi les homopolyosides, on peut tout d'abord citer les glucosanes tels que l'amidon, le glycogène, la cellulose, le dextrane, et la chitine. A côté des glucosanes, on peut également citer les arabanes, les xylanes et les pectines.

Parmi les hétéropolyosides, on peut notamment citer les gommes qui sont des structures ramifiées comportant du D-galactose, du L-arabinose, du L-rhamnose, et de l'acide D-glucuronique, et les hémicelluloses.

Le dextrane est un polysaccharide neutre issu de bactéries telles que *Leuconostoc mesenteroide* et *Leuconostoc dextranicum*. Ces bactéries possèdent une enzyme, la dextransucrase, qui transforme le saccharose en macromolécules composées d'unités D-glucose liées en $\alpha(1,6)$, comportant plusieurs fonctions hydroxyle. Les propriétés colloïdales du dextrane ainsi que sa faible immunogénicité ont permis son utilisation comme substitut du plasma. Étant donné que les groupements hydroxyles du dextrane peuvent être aisément fonctionnalisés, de nombreux dérivés du dextrane ont été préparés. En fonction de la nature des substituants et du degré de substitution, ces produits présentent des propriétés biologiques beaucoup plus étendues que celles du dextrane natif.

Dans le cas particulier des dextranes fonctionnalisés, il a par exemple été décrit que certains dérivés du dextrane carboxyméthylés puis benzylamidifiés (CMDB), en fonction de leur degré de substitution, présentaient une activité antiproliférative sur différentes lignées cellulaires, notamment sur les lignées prétumorales du sein HBL100 et HH9 et sur les cellules cancéreuses du sein humain (MCF-7), (R. Baghéri-Yarmand *et al.*, 1994, In Vitro Cell. Dev. Biol. **30A**, 8822-8824). Il a été montré que cette activité antiproliférative était liée à la présence de groupement benzylamide. L'inhibition de la croissance cellulaire est associée à une diminution du nombre de cellules en phase S et à une accumulation de cellules en phase $G_0/G_1$ (R. Baghéri-Yarmand *et al.*, Anticancer Res., 1992, **12**, 1641-1616). De récentes études ont montré que certains CMDB modifient l'interaction des différents facteurs de croissance (FGF 2, FGF 4, PDGF BB, TGFβ) avec leurs récepteurs membranaires (voir par exemple P. Bittoun *et al.*, Biochemical Pharmacology, 1999, **57**, 1399-1406). Les CMDB exercent leur activité antiproliférative en interférant avec les circuits paracrine et autocrine, (Liu JF. *et al.*, Anticancer Res., 1997, **17**, 253-258 ; Baghéri-Yarmand R. *et al.*, Biochem. Biophys. Res. Commun., 1997, **239**, 424-428; Baghéri-Yarmand R. *et al.*, Br. J. Cancer., 1998, **78**, 111-118 et Baghéri-Yarmand R. *et al.*, Cell. Growth. Differ., 1998, **9**, 497-504). Des tests *in vivo* ont démontré chez la souris athymique (ou nude) que la croissance des tumeurs induites par inoculation d'un modèle de cellules cancéreuses du sein humain MCF-7*ras* était bloquée par traitement au CMDB. L'analyse histologique a montré une diminution de la vascularisation de ces tumeurs. Il a également été montré que certains dérivés carboxyméthylés puis benzylamidifiés, puis sulfonatés (CMDBS), en fonction de leur degré de substitution, avaient également une action antiproliférative sur les lignées cellulaires HBL100 et HH9 (Baghéri-Yarmand R. *et al.*, Biochem. Biophys. Res. Commun., 1997, **239**, 424-428).

Toujours dans le but de trouver des thérapies anti-cancéreuses, il a également déjà été proposé d'utiliser certains sels de l'acide phénylacétique, tels que par exemple le phénylacétate de sodium (NaPA). Le NaPA est un métabolite commun de la phénylalanine, habituellement présent dans le plasma à des concentrations μmolaires. De plus fortes concentrations en NaPA, (de l'ordre du mmolaire), entraînent une cytostase et la réversion de plusieurs phénotypes de cellules cancéreuses humaines, (voir en particulier Samid D. *et al.*, Cancer Res., 1992, **52**, 1988-1992 ; Samid D. *et al.*, Blood, 1992, **80**, 1576-1581 ; Samid D. *et al.*, J. Clin. Invest., 1993, **91**, 2288-2295, et Samid D. *et al.*, Cancer Res., 1994, **54**, 891-895). Le NaPA et ses dérivés présentent également une activité antitumorale vis-à-vis du modèle de cellules cancéreuses MCF-7ras implantées dans la souris athymique, (Adam L. *et al.*, Cancer Res., 1995, **55**, 5156-5160). Le NaPA a été utilisé en phase I et en phase II d'essais cliniques, réalisés sur des patients présentant des tumeurs malignes de la prostate, des médulloblastomes ou des gliomes, (Thibault A. *et al.*, Cancer Res., 1994, **54**, 1690-1694 et Chang SM. *et al.*, J. Clin; Oncol., 1999, **17**, 3, 984-990). Le mécanisme par lequel le NaPA inhibe la prolifération cellulaire est réversible et dépendant de la dose administrée.

Le but d'une thérapie anticancéreuse est de tuer les cellules tumorales ou de bloquer leur prolifération, tout en épargnant les cellules normales. Pour être efficace contre les cellules tumorales, le NaPA doit être utilisé *in vivo* à des concentrations de l'ordre de 150 mg/kg. Cependant, à cette concentration, le NaPA a un effet cytotoxique aussi bien sur les cellules cancéreuses que sur les cellules saines, ce qui n'est pas souhaitable.

C'est pourquoi il existe un réel besoin de mettre au point de nouveaux traitements anticancéreux n'ayant pas d'effet cytotoxique sur les cellules saines.

Il a déjà été proposé, notamment dans le brevet US 5,710,178 d'associer le NaPA à certaines substances ayant une activité biologique telles que par exemple les interférons, les interleukines, les facteurs de nécrose tumorale, les antagonistes de glutamine, les hormones, les vitamines, etc. Ce type d'association ne permet pas de diminuer les doses de NaPA utilisées.

C'est afin de remédier à ces problèmes et de pourvoir à un nouvel anticancéreux que les Inventeurs ont mis au point ce qui fait l'objet de l'invention.

La présente invention a pour objet une composition pharmaceutique, caractérisée par le fait qu'elle comprend, à titre de principe actif, au moins une composition choisie dans le groupe constitué par :

a) un conjugué d'un polymère comportant au moins une fonction hydroxyle libre et d'au moins un dérivé d'un acide phénylalkylcarboxylique de formule (I) suivante :

$$R_2\text{-}(CH_2)_n\text{-}COOR_1 \tag{I}$$

dans laquelle :

- $R_1$ représente un atome d'hydrogène, un atome d'halogène, un atome d'un métal alcalin monovalent tel que le sodiumou le potassium, ou un groupement $-CO(CH_2)_m R_3$,
- $R_2$ et $R_3$ représentent un radical phényle non substitué,
- n et m, identiques, sont des nombres entiers compris entre 1 et 3 inclusivement ;

ledit conjugué répondant à la formule (III) suivante :

$$(\text{Polymère})\text{-}(OOC\!\!-\!\!(CH_2)_n\text{-}R_2)_d \tag{III}$$

dans laquelle le polymère comporte au moins une fonction hydroxyle libre, n est un nombre entier compris entre 1 et 3 inclusivement, $R_2$ représente un radical phényle non substitué et $\underline{d}$, qui est l'indice de substitution (ds) en dérivé de formule (I), est $\geq 0,05$ et $\leq 1,5$ et de préférence $> 0,15$ et encore plus préférentiellement compris entre 0,5 et 0,8 inclusivement ;

b) une association d'au moins un polymère présentant une masse moléculaire supérieure ou égale à 5000 Da et comportant au moins une fonction hydroxyle libre et d'au moins un sel d'un acide phénylalkylcarboxylique de formule (I') suivante :

$$R'_2\text{-}(CH_2)_{n'}\text{-}COOR'_1 \tag{I'}$$

dans laquelle :

- $R'_1$ est un atome d'un métal alcalin monovalent tel que le sodium ou le potassium,
- $R'_2$ représente un radical phényle non substitué,
- n' est un nombre entier compris entre 1 et 3 inclusivement ;

c) et leurs mélanges,

et éventuellement un ou plusieurs autres principes actifs additionnels, et en présence ou non d'un véhicule pharmaceutiquement acceptable et/ou d'un support physiologiquement acceptable.

Selon l'invention, les composés conjugués de formule (III) ci-dessus sont trivialement appelés NaPAC.

Egalement selon l'invention, le véhicule pharmaceutiquement acceptable peut être constitué par un ou plusieurs excipients et/ou par un ou plusieurs transporteurs tels que les liposomes.

Les polymères utilisés conformément à l'invention peuvent être naturels (natifs) ou synthétiques, et être éventuellement substitués (fonctionnalisés), au niveau des fonctions hydroxyles libres, par un ou plusieurs groupements chimiques fonctionnels choisis parmi les groupements carboxylique ; alkylcarboxylique tel que par exemple méthylcarboxylique ; arylalkylcarboxylique ; N-benzyléthylènecarboxamide ; sulfate ; et sulfonate ; capables de conférer à ces polymères des propriétés biologiques ou physico-chimiques supplémentaires.

Les polymères pouvant être utilisés selon la présente invention sont de préférence choisis parmi les polyols et les polysaccharides, naturels ou synthétiques, et éventuellement fonctionnalisés.

Parmi les polyols, on peut notamment citer à titre d'exemple, les polyalkylène glycols tels que le polyéthylène glycol et le polypropylène glycol, et les polyvinylalcools.

Selon une forme de réalisation avantageuse de l'invention les polymères sont choisis parmi les polysaccharides, naturels ou synthétiques, et éventuellement fonctionnalisés, parmi lesquels on peut notamment citer les glucosanes tels que l'amidon, le glycogène, les celluloses, les dextranes, les poly-β-1,3-glucanes et la chitine ; les arabanes ; les xylanes et les pectines.

Les dextranes et les poly-β-1,3-glucanes, naturels ou synthétiques, éventuellement fonctionnalisés, sont particulièrement préférés, notamment lorsque la composition pharmaceutique conforme à l'invention renferme ledit ou lesdits polymères et le ou les sel(s) de l'acide phénylalkylcarboxylique de formule (I') sous forme associée.

A titre de dextranes synthétiques fonctionnalisés, on peut notamment mentionner les composés décrits dans les demandes de brevet EP-A-0 023 854 ; EP-A-0 146 455 ; ou WO 99/29734, tels que par exemples les dextranes comportant une ou plusieurs fonctions carboxylique, méthylcarboxylique, N-benzylméthylènecarboxamide, sulfate, et/ou sulfonate.

Parmi ces dextranes synthétiques fonctionnalisés, on préfère les dérivés de dextrane de formule (II) suivante :

$$DMC_aB_bSu_c \qquad\qquad (II)$$

dans laquelle :

- D représente une chaîne polysaccharidique, de préférence constituée par des enchaînements d'unités glucosidiques,
- MC représente des groupes méthylcarboxylique,
- B représente des groupes N-benzylméthylènecarboxamide,
- Su représente des groupes sulfates (sulfatation des fonctions hydroxyle libres, portées par les unités glucosidiques),
- $a$, $b$ et $c$ représentent le degré de substitution (ds), respectivement des groupements MC, B et Su ; $a$ étant égal à 0 ou $\leq$ à 2 ; $b$ étant égal à 0 ou $\leq$ à 1 ; $c$ étant égal à 0 ou $\leq$ à 1 et ; étant entendu que la somme $a + b + c \leq$ à 3.

Les dextranes de formule (II), ainsi que leur procédé de préparation sont décrits dans la demande de brevet WO 99/29734. Ces dérivés de dextrane de formule (II) sont trivialement appelés DMCB et sont considérés comme étant des copolymères constitués de sous-unités fictives R-OX, X pouvant être un groupement méthylcarboxylique (MC), benzylamide (B) ou sulfate. La chaîne polysaccharidique est donc considérée comme étant constituée de 300 % de sous-unités fictives R-OH, au lieu de 100 % d'unités glucosidiques. Ainsi, un dextrane méthylcarboxylique (DCM) avec un degré de substitution (ds) de 1,2 en groupements méthylcarboxyliques contient 1,2 groupement substitué (R-MC) et 1,8 groupement hydroxyle libre (R-OH), par unité glucosidique.

Parmi les dérivés de dextrane de formule (II) décrits ci-dessus, on préfère les composés dans lesquels 0,5 $\leq a \leq$ 1,5 ; 0,1 $\leq b \leq$ 1 ; et 0 $\leq c \leq$ 0,6.

Parmi ces dérivés de dextrane de formule (II), on préfère encore plus particulièrement ceux dans lesquels :

i) $a$ = 0,70 ; $b$ = 0,3 ; $c$ = 0 ;
ii) $a$ = 0,91 ; $b$ = 0,18 ; $c$ = 0 ;
iii) $a$ = 0,67 ; $b$ = 0,33 ; $c$ = 0 ; (dénommé LS4 DMCB),
iv) $a$ = 0,68 ; $b$ = $c$ = 0 ; (dénommé LS12),
v) $a$ = 0,67 ; $b$ = 0,39 ; $c$ = 0 ; (dénommé LS17),
vi) $a$ = 0,68 ; $b$ = 0,34 ; $c$ = 0 ;
vii) $a$ = 0,68 ; $b$ = 0,20 ; $c$ = 0 ;
viii) $a$ = 0,7 ; $b$ = 0,3 ; $c$ = 0 ; (dénommé DMCB7),
ix) $a$ = 0,60 ; $b$ = 0,40 ; $c$ = 0 ; (dénommé LS17-DMCB),
x) $a$ = 0,60 ; $b$ = 0,35 ; $c$ = 0 ; (dénommé DMCB10).

Parmi les sels d'acide phénylalkylcarboxylique de formule (I') ci-dessus, on peut notamment citer le phénylacétate de sodium, le phénylacétate de potassium, le phénylpropionate de sodium, le phénylpropionate de potassium, le phénylbutyrate de sodium et le phénylbutyrate de potassium.

Lorsque la composition pharmaceutique conforme à l'invention renferme au moins un dextrane de formule (II) conjugué à au moins un composé de formule (I), le composé conjugué ainsi formé répond à la formule (IIIa) suivante :

$$(DMC_aB_bSu_c)(OOC\text{-}(CH_2)_nR_2)_d \qquad (IIIa)$$

dans laquelle D ; MC ; C ; B ; Su ; $R_2$ ; $\underline{a}$, $\underline{b}$, et $\underline{c}$ ont les mêmes significations que celles indiquées ci-dessus pour les composés de formules (I) et (II) et $\underline{d}$ a la même signification que celle indiquée ci-dessus pour les composés de formule (III), la somme $\underline{a}$ + $\underline{b}$ + $\underline{c}$ + $\underline{d}$ étant inférieure ou égale à 3.

Selon une forme de réalisation particulièrement préférée, la composition pharmaceutique conforme à l'invention renferme au moins un composé conjugué de formule (III) décrite ci-dessus, et encore plus préférentiellement au moins un composé conjugué de formule (IIIa).

Parmi les composés conjugués de formule (IIIa) ci-dessus, on préfère ceux dans lesquels $\underline{a}$ > 0,5 ; $\underline{b}$ > 0,15 ; $\underline{c}$ = 0 ou < 0,8 et $\underline{d}$ < 1,5.

Encore plus particulièrement, on préfère les composés conjugués de formule (IIIa) dans lesquels :

i) $\underline{a}$ = 0,68 ; $\underline{b}$ = 0,20 ; $\underline{c}$ = 0 et $\underline{d}$ = 0,14 ;
ii) $\underline{a}$ = 0,64 ; $\underline{b}$ = 0,43 ; $\underline{c}$ = 0 et $\underline{d}$ = 0,35 ;
iii) $\underline{a}$ = 0,67 ; $\underline{b}$ = 0,39 ; $\underline{c}$ = 0 et $\underline{d}$ = 0,35 (LS17-NaPAC) ;
iv) $\underline{a}$ = 1 ; $\underline{b}$ = $\underline{c}$ = 0 et $\underline{d}$ = 0,1 ; et
v) $\underline{a}$ = 1 ; $\underline{b}$ = $\underline{c}$ = 0 et $\underline{d}$ = 0,3.

Le ou les polymères associés à au moins un sel d'un acide phénylalkylcarboxylique de formule (I') et les composés conjugués de formules (III) ou (IIIa) présentent de manière surprenante une activité anti-cancéreuse supérieure à celle de chacun des composés utilisés séparément permettant par conséquent de minuer les doses efficaces en chacun des principes actifs, tout en augmentant efficacité anticancéreuse de la composition pharmaceutique les contenant.

Également de manière surprenante, cette activité anti-cancéreuse est encore plus importante lorsqu'un composé conjugué de formule (III) ou (IIIa) est utilisé.

Lorsque le ou les polymères et le ou les sels d'un acide phénylalkylcarboxylique de formule (I') sont présents au sein de la composition pharmaceutique conforme à l'invention sous la forme associée, alors le rapport de concentrations (exprimées en mM) entre le ou les polymères d'une part et le ou les composés de formule (I') d'autre part est de préférence compris entre 10:1 et 1:1, et encore plus préférentiellement entre 10:2 et 1:1.

La composition pharmaceutique conforme à l'invention peut bien entendu renfermer un ou plusieurs additifs classiquement utilisés pour la préparation de compositions pharmaceutiques, tels que des anti-agglomérants, des anti-oxydants, des colorants, des agents modifiants du goût, des agents de lissage, de montage, d'isolation et de manière générale, tout excipient classiquement utilisé dans l'industrie pharmaceutique.

Les compositions pharmaceutiques conformes à l'invention présentent un certain nombre d'activités biologiques : antiprolifératives, cytostatiques, nécrosantes, pro-apoptotiques, anti-angiogéniques, anti-métastatiques et inhibitrices des facteurs mitogéniques.

Elles sont donc particulièrement actives comme anticancéreux, notamment sur les cancers du sein (hormone-dépendants ou non-hormono-dépendants) et sur les mélanomes.

Ladite composition pharmaceutique est de préférence administrée par voie systémique, telle que les voies sous-cutanée, intraveineuse et orale.

Les doses efficaces globales du ou des polymères associés à au moins un sel d'un acide phénylalkylcarboxylique de formule (I'), ou de composés conjugués de formule (III), peuvent varier dans de larges proportions allant de préférence de 0,1 mg/kg par jour à 200 mg/kg par jour, à raison de deux fois par semaine et encore plus préférentiellement de 15 mg/kg par jour à 150 mg/kg par jour, à raison de deux fois par semaine, notamment lorsqu'un composé conjugué de formule (III) est utilisé.

Les composés conjugués de formule (III) tels que décrits ci-dessus sont des composés nouveaux qui constituent à ce titre un autre objet de l'invention. Parmi les composés de formule (III), les composés de formule (IIIa) sont préférés.

Parmi les composés de formule (IIIa), on préfère ceux dans lesquels $\underline{a}$ > 0,5 ; $\underline{b}$ > 0,15 ; $\underline{c}$ = 0 ou < 0,8 et $\underline{d}$ < 1,5.

Encore plus particulièrement, on préfère les composés conjugués de formule (IIIa) dans lesquels les ds sont les suivants :

i) $\underline{a}$ = 0,68 ; $\underline{b}$ = 0,20 ; $\underline{c}$ = 0 et $\underline{d}$ = 0,14 ;

ii) $\underline{a}$ = 0,64 ; $\underline{b}$ = 0,43 ; $\underline{c}$ = 0 et $\underline{d}$ = 0,35 ;
iii) $\underline{a}$ = 0,67 ; $\underline{b}$ = 0,39 ; $\underline{c}$ = 0 et $\underline{d}$ = 0,35 (LS17-NaPAC) ;
iv) $\underline{a}$ = 1 ; $\underline{b}$ = $\underline{c}$ = 0 et $\underline{d}$ = 0,1 ; et
v) $\underline{a}$ = 1 ; $\underline{b}$ = $\underline{c}$ = 0 et $\underline{d}$ = 0,3.

sont particulièrement préférés.

Les composés conjugués de formule (III) tels que décrits ci-dessus peuvent être préparés selon les méthodes classiques d'estérification consistant par exemple à réaliser, dans un milieu solvant organique, une réaction d'estérification entre au moins une fonction hydroxyle libre du polymère et au moins un dérivé d'un acide phénylalkylcarboxylique de formule (I) telle que décrite ci-dessus, en présence d'une amine tertiaire aliphatique ou aromatique ou d'un agent de couplage ou d'un chlorure d'acyle ou d'un anhydride, pour former une liaison covalente entre ledit dérivé de formule (I) et ledit polymère et conduire ainsi au composé de formule (III) attendu.

Ce procédé de préparation peut comporter une étape préliminaire de solubilisation du polymère par exemple soit par chauffage dudit polymère dans le solvant de réaction en présence de sels de lithium, soit, uniquement dans le cas des dextranes fonctionnalisés, par échange d'ions sur colonne et neutralisation par une amine tertiaire aliphatique ou aromatique de préférence celle utilisée comme catalyseur dans la réaction d'estérification.

Les facteurs pouvant influencer l'estérification sont au nombre de cinq :

1) la nature du solvant organique : ils sont de préférence choisis parmi le diméthylsulfoxide (DMSO), le formamide, le diméthylacétamide, le diméthylformamide (DMF), et leurs mélanges. Le DMF est particulièrement préféré ;

2) la nature de l'amine tertiaire ou de l'agent de couplage : les amines tertiaires pouvant être utilisées selon ce procédé, sont de préférence choisies parmi la pyridine et ses dérivés alkylés, la triéthylamine, la tributylamine, et la N,N-diméthylaniline. La pyridine et la triéthylamine sont particulièrement préférées. La quantité d'amine tertiaire à utiliser peut être calculée en fonction de la quantité de fonctions hydroxyles libres dans le polymère utilisé. A titre d'agent de couplage, on peut par exemple utiliser une carbodiimide telle que le 1,1'-carbonyldiimidazole (CDI) ou la N,N'-dicyclohexylcarbodiimide (DCC), à une température comprise entre 0 et 60°C, le rapport molaire entre le dérivé d'un acide phénylalkylcarboxylique de formule (I) et la CDI étant compris entre 0,001 et 10, et le rapport molaire entre la CDI et le polymère pouvant varier entre 0,01 et 10. La CDI est soluble dans les solvants organiques tels que le DMSO, la DMF, le formamide, etc.,

3) le temps de réaction ;
4) la température de la réaction ;
5) le nombre (ou concentration) en fonctions hydroxyles libres dans le polymère utilisé.

A titre d'exemple, la quantité de fonctions hydroxyles libres dans les composés de formule (II), sous forme de sel de triéthylamine, peut être calculée selon la méthode suivante :

On considère que chaque unité glucosidique est constituée de trois sous-unités fictives substituables. On a donc :

R-OH        Masse Molaire : 54g/mol
$ROCH_2CONHCH_2\varphi$ ($\varphi$ = phényle)        Masse Molaire : 201g/mol
$ROCH_2COOHNEt_3$ (Et = éthyle)        Masse Molaire : 213 g/mol

En fonction de la masse du produit sec, le nombre de fonctions hydroxyles libres est déterminé grâce aux équations suivantes :

$$(1) \qquad h \times 0{,}054 + b \times 0{,}201 + t \times 0{,}213 = 1g$$

avec

$$[h/(h+b+t)] \times 3 = ds_{OH}$$

$$[b/(h+b+t)] \times 3 = ds_{\underline{b}}$$

$$[t/(h+b+t)] \times 3 = ds_{\underline{a}}$$

h : nombre de mole d'hydroxyles libres sur le dérivé de dextrane ;

b : nombre de mole de groupements N-benzylméthylènecarboxamide ($ROCH_2CONHCH_2\varphi$) sur le composé de formule (II) ;

t : nombre de mole de groupements $ROCH_2COOHNEt_3$ sur le composé de formule (II) ;

$ds_{\underline{b}}$ : degré de substitution en groupements N-benzylméthylènecarboxamide ;

$ds_{\underline{a}}$ : degré de substitution en groupements méthylcarboxylique ;

$ds_{OH}$ : taux de OH libre.

Soit

$$(2): \quad h+b+t = (3 \times h)/ds$$

Ce qui donne :

$$t = ds_{\underline{a}}/ds_{OH}$$

$$b = ds_{\underline{b}}/ds_{OH}$$

on remplace alors ces équations dans l'équation (1) :

$$h[0,054+(ds_{\underline{a}}/ds_{OH}) \times 0,213+(ds_{\underline{b}}/ds_{OH}) \times 0,201] = 1$$

par conséquent,

$$h = 1/[0,054+(ds_{\underline{a}}/ds_{OH}) \times 0,213+(ds_{\underline{b}}/ds_{OH}) \times 0,201]$$

Par exemple, pour un composé de formule (II) présentant les degrés de substitution suivants : $ds_{\underline{b}} = 0,33$ ; $ds_{\underline{a}} = 0,67$ ; $ds_{OH} = 3-(ds_{\underline{b}} + ds_{\underline{a}}) = 2$

La quantité de résidus hydroxyles libres est donc h = 6,06 mmole/g

Les volumes de triéthylamine (V1) et du dérivé d'un acide phénylalkylcarboxylique de formule (I) utilisé dans l'étape de conjugaison (V2) sont déterminés en fonction des rapports suivants :

V1 : $(N_{\varphi CH2COCl}/N_{OH})$     V2 : $(N_{\varphi CH2COCl}/N_{NET3})$

avec

$N_{\varphi CH2COCl}$ : nombre de mmole de dérivé d'un acide phénylalkylcarboxylique de formule (I)

$N_{OH}$ : nombre de mmole de groupement hydroxyle.

$N_{NET3}$ : nombre de mmole de triéthylamine.

Dans les composés conjugués de formule (III), les degrés de substitution $\underline{d}$ en composés de formule (I) peuvent être déterminés par dosage acidimétrique, spectrophotométrie infra rouge ou spectrophotométrie d'absorption UV.

Lorsque la synthèse est terminée, les composés conjugués de formule (III) peuvent éventuellement être purifiés selon des méthodes classiques telles que par exemple la précipitation en milieu alcoolique et/ou l'ultrafiltration.

Outre les dispositions qui précèdent, l'invention comprend encore d'autres dispositions qui ressortiront de la description qui va suivre, qui se réfère à des exemples de synthèse de composés conjugués de formule (III) selon l'invention et à plusieurs exemples de démonstration de la synergie d'action entre un dextrane de formule (II) associé ou conjugué à un sel d'un acide phénylalkylcarboxylique de formule (I), ainsi qu'aux figures annexées, dans lesquelles:

- la figure 1 représente les effets inhibiteurs comparés des produits seuls et des produits associés sur la prolifération des cellules MCF-7 ;
- la figure 2 montre les effets du NaPa en association avec le LS4 DMCB sur la répartition des cellules MCF-7*ras* dans les trois phases du cycle cellulaire ($G_0/G_1$/S et $G_2$/M) ;
- la figure 3 représente les effets comparés des produits seuls et des produits associés sur la diminution du volume des tumeurs induites par inoculation de cellules MCF-7*ras* chez la souris athymique ;
- la figure 4 représente les effets d'un composé conjugué de formule (III) (NaPAC) sur la diminution du volume des tumeurs induites par inoculation de cellules MCF-7*ras* chez la souris athymique ; et
- la figure 5 montre les effets du NaPA seul et du LS17-NaPAC sur la diminution du volume des tumeurs induites par inoculation de cellules 1205LU chez la souris athymique ;

Il doit être bien entendu toutefois que ces exemples sont donnés uniquement à titre d'illustration de l'objet de l'invention, dont ils ne constituent en aucune manière une limitation.

## EXEMPLE 1 : SYNTHÈSE du LS17-NaPAC

La synthèse du LS17-NaPAC a nécessité tout d'abord la mise sous forme de sel de pyridine du LS17 précurseur afin de pouvoir le solubiliser dans un solvant polaire anhydre, généralement une base de Lewis, tel que le DMF.

### Mise sous forme de sel de pyridine

41 g de LS17 ont été dissous dans 774 ml d'eau bidistillée. La solution ainsi obtenue a été versée à travers une colonne contenant 69,2 g de résine d'échange d'ion Purolite C100H. Le pH de la solution récupérée en sortie de colonne de résine a été ajusté à 5.5 à l'aide de la pyridine. Cette solution a alors été congelée et lyophilisée. On a obtenu environ 38 g de LS 17 sous forme de sel de pyridine (LS 17-pyr) après séchage à l'étuve sous vide à 50°C pendant 24h.

### Fixation du phénylacétate

37g de LS17-pyr obtenus ci-dessus à l'étape précédente ont été dissous dans 2,5 L de DMF anhydre. La solution obtenue a été versée dans un réacteur de 5L double enveloppe sous azote. 42,8 ml de pyridine y ont ensuite été ajoutés. Par ailleurs, une solution de chlorure d'acide phénylacétique a été préparée en mélangeant 35,2 ml de chlorure d'acide phénylacétique dans 352 ml de DMF anhydre et ajoutée rapidement au mélange du réacteur double enveloppe sous agitation. Le mélange réactionnel a été laissé sous agitation pendant 1 heure.

La réaction a été arrêtée par ajout d'une solution 1M de soude. Le LS17-NaPAC obtenu a alors été purifié par ultrafiltration tangentielle à l'aide d'un système Millipore muni d'une membrane de seuil de coupure 5000 g/mol. La solution purifiée a alors été concentrée, congelée puis lyophilisée.

Le LS17-NaPAC présentait les degrés de substitution suivants :

$\underline{a}$ = 0,67 ; $\underline{b}$ = 0,39 ; $\underline{c}$ = 0 et $\underline{d}$ = 0,35.

## EXEMPLES 2 A 15 : SYNTHÈSE DE COMPOSÉS CONJUGUÉS DE FORMULE (III) DANS LE DMF

Dérivé de dextrane utilisé : LS4 DMCB dans lequel $\underline{a}$ = 0,67 et $\underline{b}$ = 0,33

### Étape préalable de solubilisation du LS4 DMCB

Le dextrane LS4 DMCB a été solubilisé, sous la forme de son sel d'amine tertiaire, par chromatographie échangeuse de cations (résine IR, 120, H$^+$). 4 amines ont été utilisées : la triéthylamine, la tributylamine, la pyridine et la N,N-diméthylaniline.

6 g de dérivé de dextrane LS4 DMCB ont été dissous dans 200 ml d'eau bidistillée, puis chromatographiés sur une colonne de résine IR 120 H$^+$ (volume : 1,9 méq/ml ; capacité : 10%). Le produit a été chromatographié avec un volume d'eau bidistillée représentant deux à trois fois le volume de la colonne. Le produit récupéré a été neutralisé par une solution d'amine tertiaire. Cette dernière a été préparée différemment en fonction de l'amine tertiaire utilisée. La tributylamine et la N,N-diméthylamine n'étant pas miscibles dans l'eau, la solution de neutralisation a été préparée dans l'éthanol. La solution finale d'un volume de 1000 ml a été concentrée par évaporation rotative puis congelée et lyophilisée.

Le dextrane LS4 DMCB ainsi solubilisé a été utilisé pour préparer les composés conjugués de formule (III) appelés ci-après NaPAC MF.

Les conditions de synthèse sont résumées dans le tableau I ci-après. Avant toute synthèse, le dérivé de dextrane est placé dans une étuve à 50°C. Le dextrane séché est dissous dans le DMF sous agitation dans un réacteur à double paroi maintenu à la température de la réaction. Une solution de l'amine tertiaire à 10% (v : v) dans le DMF est ajoutée à l'aide d'une seringue. Le chlorure de l'acide phénylacétique, préparé de la même manière, est alors ajouté à l'aide d'une seringue. Au bout d'une heure, la réaction est stoppée par l'ajout de 150 ml d'eau bidistillée. Le pH est ajusté à 7 à l'aide d'une solution de soude 0,1 M.

La solution obtenue est purifiée en trois étapes :

- Évaporation à sec à l'évaporateur rotatif pour éliminer les solvants ;

- Extraction à l'éther en milieu basique : le solide obtenu après l'évaporation est redissous dans 50 ml d'eau bidistillée. Le pH de la solution ainsi obtenue est ajusté à 10 à l'aide d'une solution de soude diluée puis la solution est lavée avec de l'éther diéthylique (20 ml x 3) dans une ampoule à décanter. La phase aqueuse est neutralisée avec de l'acide chlorhydrique dilué, puis ultrafiltrée ;
- Ultrafiltration : le volume de la phase aqueuse est ajusté à 2 litres avec de l'eau bidistillée, ultrafiltrée avec une solution de NaCl 2M (20L) puis avec de l'eau bidistillée. Le rétentat est congelé puis lyophilisé.

Les produits des exemples 2 à 9, 11 et 15 ont été purifiés et après purification, les produits obtenus présentaient les caractéristiques suivantes :

| Exemple | Composé conjugué de formule (III) | Rendement | Degré de substitution en NaPA (d) |
|---------|-----------------------------------|-----------|-----------------------------------|
| 2 | NaPAC MF3 | 77% | $0,24 \pm 0,05$ |
| 3 | NaPAC MF4 | 82% | $0,68 \pm 0,1$ |
| 4 | NaPAC MF5 | 70% | $0,07 \pm 0,04$ |
| 5 | NaPAC MF6 | 91% | $0,71 \pm 0,1$ |
| 6 | NaPAC MF7 | 71% | $0,43 \pm 0,05$ |
| 7 | NaPAC MF8 | 84% | $0,53 \pm 0,08$ |
| 8 | NaPAC MF9 | 81% | Non calculable |
| 9 | NaPAC MF10 | 95% | $0,22 \pm 0,09$ |
| 11 | NaPAC MF12 | 90% | $0,27 \pm 0,01$ |
| 15 | NaPAC MF14 | 79% | $0,28 \pm 0,06$ |

## TABLEAU I : CONDITIONS DE SYNTHÈSE DANS LE DMF

| Ex. | Composé de formule (III) | Amine tertiaire | Tempé rature | Concen-tration en OH libre | Quantité OH libre en mole | Quantité RCOCl en mole | Quantité Net3 en mole V1 | V1 | V2 | Volume * RCOCl en ml | Volume * d'amine en ml | Volume DMF en ml | Volume final |
|---|---|---|---|---|---|---|---|---|---|---|---|---|---|
| 2 | NaPAC MF3 | Pyridine | 30°C | 0,12 M | $5{,}87.10^{-3}$ | $5{,}87.10^{-3}$ | $1{,}17.10^{-2}$ | 1 | 2 | 7,8 | 9,8 | 31,3 | 48,9 |
| 3 | NaPAC MF4 | Pyridine | 30°C | 0,06 M | $5{,}93.10^{-3}$ | $5{,}93.10^{-3}$ | $1{,}19.10^{-2}$ | 1 | 2 | 7,9 | 9,8 | 81,1 | 98,8 |
| 4 | NaPAC MF5 | Triéthylamine | 30°C | 0,12 M | $6{,}90.10^{-3}$ | $6{,}90.10^{-3}$ | $1{,}38.10^{-2}$ | 1 | 2 | 9,2 | 18,9 | 29,4 | 57,5 |
| 5 | NaPAC MF6 | Triéthylamine | 30°C | 0,06 M | $6{,}24.10^{-3}$ | $6{,}24.10^{-3}$ | $1{,}25.10^{-2}$ | 1 | 2 | 8,2 | 17,0 | 104 | 129,2 |
| 6 | NaPAC MF7 | Triéthylamine | 30°C | 0,12 M | $4{,}93.10^{-3}$ | $4{,}93.10^{-3}$ | $9{,}86.10^{-3}$ | 1 | 2 | 6,5 | 23,5 | 11,0 | 41,0 |
| 7 | NaPAC MF8 | Triéthylamine | 30°C | 0,06 M | $4{,}83.10^{-3}$ | $4{,}83.10^{-3}$ | $9{,}66.10^{-3}$ | 1 | 2 | 6,4 | 23,0 | 51,0 | 80,4 |
| 8 | NaPAC MF9 | N,N-diéthylamine | 30°C | 0,12 M | $3{,}73.10^{-3}$ | $3{,}73.10^{-3}$ | $7{,}46.10^{-3}$ | 1 | 2 | 5,0 | 9,5 | 16,6 | 31,1 |
| 9 | NaPAC MF10 | N,N-diéthylamine | 30°C | 0,06 M | $4{,}42.10^{-3}$ | $4{,}42.10^{-3}$ | $8{,}84.10^{-3}$ | 1 | 2 | 5,8 | 16,8 | 51,0 | 73,6 |
| 10 | NaPAC MF11 | Pyridine | 50°C | 0,12 M | $5{,}93.10^{-3}$ | $5{,}93.10^{-3}$ | $1{,}19.10^{-2}$ | 1 | 2 | 8,0 | 9,8 | 51,3 | 69,1 |
| 11 | NaPAC MF12 | Pyridine | 50°C | 0,06 M | $5{,}83.10^{-3}$ | $5{,}83.10^{-3}$ | $1{,}17.10^{-2}$ | 1 | 2 | 7,7 | 9,6 | 79,8 | 97,1 |
| 12 | NaPAC MF13 | Triéthylamine | 50°C | 0,12 M | $6{,}11.10^{-3}$ | $6{,}11.10^{-3}$ | $1{,}22.10^{-2}$ | 1 | 2 | 8,1 | 16,7 | 26,1 | 50,9 |
| 13 | NaPAC MF14 | Triéthylamine | 50°C | 0,06 M | $6{,}65.10^{-3}$ | $6{,}65.10^{-3}$ | $1{,}33.10^{-2}$ | 1 | 2 | 8,8 | 18,1 | 84,0 | 110,9 |
| 14 | NaPAC MF15 | Tributylamine | 50°C | 0,12 M | $4{,}93.10^{-3}$ | $4{,}93.10^{-3}$ | $9{,}86.10^{-3}$ | 1 | 2 | 6,5 | 23,5 | 11,0 | 41,0 |
| 15 | NaPAC MF16 | Tributylamine | 50°C | 0,06 M | $4{,}88.10^{-3}$ | $4{,}88.10^{-3}$ | $9{,}76.10^{-3}$ | 1 | 2 | 6,4 | 23,3 | 51,6 | 81,3 |

\* : solution à 10% (dans le DMF)  
V1 : (RCOCl)/[OH)  
V2 : : (RCOCl]/[amine)

MM (RCOCl) : 154,6 g/mol  
MM (NEt3) : 101,18 g/mol  
MM (pyridine) : 79,1 g/mol  
MM (N,N-diéthylamine) : 121,8 g /mol

d (RCOCl) : 1,1595 g/ml  
d (NEt3) : 0,724 g/ml  
d (pyridine) : 0,978 g/ml  
d (N,N-diéthylamine) : 0,956 g :ml

EP 1 289 515 B1

**EXEMPLE 16: DÉMONSTRATION DE LA SYNERGIE D'ACTION *IN VITRO* ENTRE UN DEXTRANE DE FORMULE (II) ASSOCIE OU CONJUGUE À UN SEL DE L'ACIDE PHÉNYLALKYLCARBOXYLIQUE DE FORMULE (I') SUR L'INHIBITION DE LA PROLIFÉRATION DES CELLULES MCF-7**

**I) Matériel et méthodes**

1. La lignée cellulaire tumorale MCF-7.

La lignée tumorale MCF-7 est une lignée de cellules épithéliales oestrogéno-sensibles provenant de métastases pleurales d'un adénocarcinome canalaire infiltrant du sein. Elles sont cultivées en milieu Dubelco Modified Eagle Medium (DMEM), additionné de 10% de sérum de veau foetal (SVF), de 1% de glutamine et de 1% de pyruvate de sodium. Les cellules sont conservées dans des cryotubes sous forme congelée dans l'azote liquide. Les cryotubes sont partiellement décongelés dans un bain-marie à 37°C. 2 ml de milieu de culture complet sont ajoutés puis la suspension cellulaire est mélangée. Au préalable, des flacons de culture (T25 COSTAR ®) sont préparés avec du milieu de culture. La suspension cellulaire est ajoutée dans les flasks et placée à l'incubateur à 37°C avec 5% de $CO_2$.
Lorsque les cellules sont pratiquement à confluence, la monocouche est lavée avec 2 ml de tampon phosphate (PBS), une fois sans calcium, ni magnésium. Une fois le PBS enlevé, 2 ml de trypsine sont déposés sur la monocouche afin de détacher les cellules. Chaque trypsination correspond à un passage numéroté. Suite à cela, 8 ml de milieu de culture complet sont ajoutés, ceci afin d'arrêter l'action de la trypsine.
Après homogénéisation, la suspension cellulaire est centrifugée pendant 3 à 4 minutes à 1000 tours par minutes. Le culot contenant les cellules est récupéré puis dilué dans 10 ml de milieu de culture complet. Le nombre de cellules par ml est déterminé à l'aide d'une cellule de Mallassez afin d'ensemencer un volume précis de cellules dans les flasks de culture 75 cm$^2$ (T75, COSTAR ®). Les cellules sont cultivées à 37°C en atmosphère humide contenant 5% de $CO_2$.

2. Cinétique de prolifération

Les cellules sont ensemencées aléatoirement à raison de 15 000 cellules par ml et par puits, ceci dans des plaques de 24 puits (COSTAR ®). Des prélèvements journaliers sont effectués à raison de 4 puits par jour. Le nombre de cellules contenues dans chacun des puits est déterminé par un comptage Coulter Counter ZM ® de manière à avoir trois mesures par puits. A partir de ces données une courbe de prolifération des cellules MCF-7 est tracée en fonction du temps.

3. Test de croissance cellulaire

Les cellules prélevées en phase exponentielle de croissance sont ensemencées dans des plaques de 24 puits à raison de 15 000 cellules par ml et par puits, ceci dans un milieu DMEM additionné de 10% de SVF, 1% de pyruvate de sodium et 1% de glutamine.
Après 24 heures d'incubation, les différentes concentrations de produits à tester sont ajoutées à raison de 4 puits par concentration. Ces produits sont dilués dans un milieu DMEM à 2% SVF, 1% de pyruvate de sodium et 1% de glutamine, de manière à obtenir les concentrations désirées. Un témoin négatif est réalisé sans addition de produit.
Après 72 heures d'incubation, les cellules sont détachées puis comptées à l'aide d'un Coulter Counter ZM ® réglé sur les paramètres prédéfinis pour les cellules MCF-7. Le diamètre (seuil) de détection étant de 5 pour une atténuation de 32. Ce dernier détermine le nombre de particules de diamètre recherché contenues dans l'échantillon. En tenant compte de la dilution effectuée, on détermine à partir de cette valeur, le nombre de cellules par ml de suspension cellulaire initiale. Le pourcentage d'inhibition I peut alors être déterminé à partir de ces résultats à l'aide de l'équation suivante :

$$\%I = [1-(\text{accroissement net des cellules traitées/accroissement net des cellules non traitées})] \times 100$$

L'accroissement net des cellules non traitées correspond au nombre de cellules contenues dans les puits témoins (milieu DMEM à 2% SVF) soustrait au nombre de cellules en $J_0$ (nombre de cellules lors de l'ajout des produits dans les puits).
L'accroissement net des cellules traitées correspond au nombre de cellules traitées à $J_{72}$ soustrait au nombre de cellules en $J_0$.

4. Test de croissance au MTT

Le MTT (bromure de 3-(4,5-diméthylthiazol-2-yl)-2,5-diphényltetrazolium) a la propriété de pouvoir être réduit en milieu alcalin, donnant ainsi un composé coloré, le bleu de formazan. Les mitochondries, grâce à leurs déshydrogénases membranaires, sont capables de réaliser cette réduction. De ce fait, le nombre de mitochondries fonctionnelles et indirectement le nombre de cellules vivantes peut être quantifié en fonction de l'intensité de la coloration.

Les cellules prélevées en phase exponentielle de croissance sont ensemencées dans des plaques de 96 puits à raison de 5 000 cellules pour 200 µl, ceci dans un milieu DMEM additionné de 2% de SVF, 1% de glutamine et 1% de pyruvate de sodium. Après 24 heures d'incubation, les différentes concentrations de produits à tester sont ajoutées à raison de 3 puits par concentration. Un témoin négatif est réalisé sans addition de produit.

Après 72 heures d'incubation, les cellules sont lavées deux fois avec une solution saline tamponnée puis 100 µl de MTT sont ajoutés dans chaque puits. Après une incubation de 4 heures, 100 µl de DMSO sont ajoutés dans les puits avant de lire la plaque à 570 nm à l'aide d'un lecteur de plaques.

Le pourcentage d'inhibition I est déterminé à partir de l'équation décrite précédemment. L'accroissement net des cellules non traitées correspond, cette fois, à la densité cellulaire contenue dans les puits témoins soustrait à celle du $J_0$. L'accroissement net des cellules traitées, quant à lui correspond à la densité cellulaire traitée à $J_{72}$ soustraite à celle du $J_0$.

## II) Résultats

1. Cinétique de prolifération des cellules MCF-7

Cette cinétique permet de caractériser la croissance des cellules MCF-7. Cette dernière, tracée en coordonnées semi-logarithmiques se décompose en trois phases :

**La phase de latence** : elle correspond au temps nécessaire aux cellules pour s'adapter à ce nouveau milieu et ainsi leur permettre d'adhérer à la surface. Cette phase est caractérisée par le temps de latence ($T_L$).

**La phase exponentielle** : elle correspond au temps nécessaire à une cellule pour se diviser par mitose et ainsi donner deux cellules filles. Cette phase est caractérisée par un temps de doublement ($T_D$) déterminé grâce à l'équation suivante :

$$T_D = [(t-t_0) \times \log 2] / (\log N - \log N_0)$$

**La phase de croissance ralentie** : elle représente les cellules à confluence. Les cellules occupent toute la surface dont elles disposent et ne peuvent plus croître. Un appauvrissement du milieu et une augmentation de la concentration des métabolites aboutit à l'apoptose.

Le temps de doublement ainsi que le temps de latence ne sont pas identiques pour toutes les cellules. En ce qui concerne les cellules MCF-7, le temps de doublement est très rapide, de l'ordre de 18 heures, le temps de latence, quant à lui, est de l'ordre de 24 heures.

2. Effets des produits à tester sur la lignée MCF-7

Ces essais ont pour but de démontrer que l'association ou la conjugaison d'au moins un dextrane et d'au moins un sel de l'acide phénylalkylcarboxylique de formule (I'), se traduit par une synergie d'action par rapport à l'activité de chacun des produits utilisés seuls. Pour cela, les produits seuls (phénylacétate de sodium : NaPA et Dérivé de dextrane LS4 DMCB), ainsi que les produits associés et conjugués correspondants ont été testés sur la lignée de cellules tumorales MCF-7. L'association consiste en un mélange du dextrane LS4 DMCB avec du NaPA dans une même solution. La conjugaison représente, quant à elle, la fixation du NaPA sur le dextrane LS4 DMCB par l'intermédiaire d'une liaison covalente. Les composés conjugués des exemples de synthèse 2, 5 et 7 (NaPAC MF3 : d = 0,24, NaPAC MF6 : d = 0,71, et NaPAC MF8: d = 0,53) ont ainsi été testés.

**a) Effet du NaPA seul**

L'effet du NaPA seul sur les cellules MCF-7 a tout d'abord été observé par comptage cellulaire, sur une gamme de concentrations allant de 1 mM à 50 mM. Le NaPA induit un effet antiprolifératif qui augmente en fonction de la concentration. Lorsque ces dernières sont supérieures à 30 mM, l'inhibition de la prolifération cellulaire dépasse les 100% traduisant ainsi un effet cytotoxique du produit. L'$IC_{50}$ (dose minimale entraînant l'arrêt de la prolifération de 50% des cellules) est obtenue pour une concentration de 5 mM.

L'effet du NaPA à des concentrations inférieures à 1 mM a été évalué. Pour des raisons pratiques, le pourcentage d'inhibition a été quantifié par la méthode colorimétrique (MTT). A ces concentrations, le NaPA ne semble pas avoir d'effet significatif sur les cellules MCF-7.

**b) Effet du dextrane LS4 DMCB seul**

L'effet inhibiteur de la prolifération, pour des concentrations s'échelonnant de 1 µg/ml à 1500 µg/ml, a été évalué par comptage direct des cellules au Coulter Counter ZM ®. Ce produit ne semble pas avoir d'effet inhibiteur sur les cellules MCF-7*ras*, ceci pour des concentrations n'excédant pas 1000 µg/ml.

Pour des concentrations supérieures, les expériences réalisées par la méthode colorimétrique au MTT, montrent que le pourcentage d'inhibition atteint un plateau avec un maximum au voisinage de 30 % d'inhibition.

**c) Effets comparés des produits seuls et des produits associés**

Les expériences comparées sont réalisées avec la méthode colorimétrique au MTT.

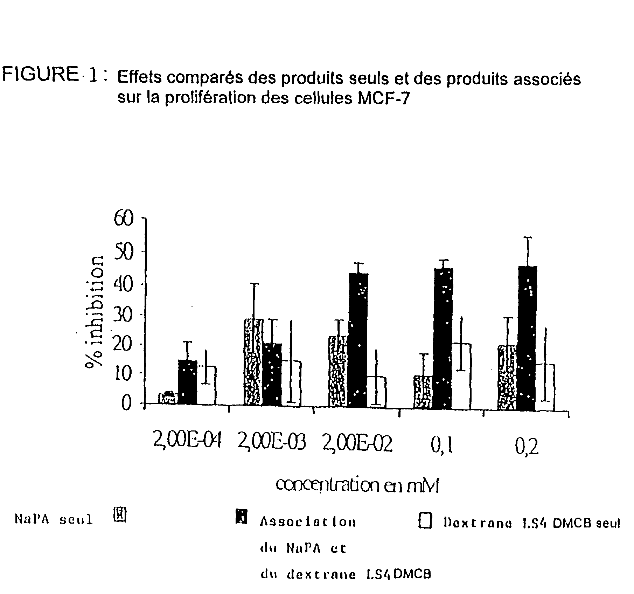

L'association du dextrane LS4 DMCB à 1000 µg/ml avec des concentrations en NaPA allant de 0,2 µM à 0,2 mM (qui correspond à des concentrations en NaPA lorsqu'elles sont exprimées en poids de 0,33 µg à 0,33 mg), permet d'obtenir une inhibition entre 40 et 50 % de la prolifération des cellules tumorales MCF-7 (figure 1). Cette même inhibition est obtenue pour une concentration en NaPA libre de 5 mM. L'association d'au moins un dextrane et d'au moins un sel d'un acide phénylalkylcarboxylique de formule (I) conforme à l'invention présente donc une synergie d'action sur l'inhibition de la prolifération des cellules MCF-7 permettant de réduire d'au moins 6 fois la quantité de NaPA utilisé.

**d) Effets comparés des produits seuls et des produits conjugués**

Les expériences comparées sont réalisées par comptage cellulaire au Coulter Counter ZM.

Tout comme pour la comparaison c), l'effet des produits conjugués sur l'inhibition de la prolifération des cellules MCF-7 est beaucoup plus important que les effets des produits utilisés seuls.

Le NaPAC MF6 de degré de substitution de 0,8 contient 2,27 mM de NaPA par mg de produit. L'effet maximal du NaPAC MF6 est observé pour une concentration de 250 µg/ml. Ceci représente une concentration équivalente de 0,56 mM de NaPA libre.

Avec le NaPA seul, on obtient cette inhibition pour une concentration de 50 mM.

Des résultats identiques ont été obtenus avec les NaPAC MF3 et NaPAC MF8.

Par conséquent, la conjugaison d'au moins un dextrane de formule (II) et d'au moins un dérivé d'un acide phénylalkylcarboxylique de formule (I) conforme à l'invention présente une synergie d'action sur l'inhibition de la prolifération des cellules MCF-7 qui permet de diminuer d'au moins 10 fois la concentration de NaPA nécessaire pour inhiber significativement la croissance des cellules MCF-7.

**EXEMPLE 17 : DÉMONSTRATION DE LA SYNERGIE D'ACTION ENTRE UN DEXTRANE DE FORMULE (II) ET UN SEL D'UN ACIDE PHÉNYLALKYLCARBOXYLIQUE DE FORMULE (I') SUR L'INHIBITION DE LA PROLIFÉRATION DES CELLULES MCF-7*ras* IN VITRO**

**I) Matériel et méthodes**

**1 Modèle de cellules cancéreuses du sein humain non-hormono-dépendant**

Des cellules MCF-7 transfectées par l'oncogène *Ha-ras* (cellules MCF-7*ras*) ont été cultivées en milieu DMEM, additionné de 10% de SVF, 2 mM de L-glutamine, 1 mM de pyruvate de sodium, 50 unités/ml de pénicilline et 50 µg/ml de streptomycine, à une température de 37°C et en atmosphère humide avec 5% de $CO_2$.

2. Test de croissance au MTT

Il a été réalisé dans des micro plaques de 96 puits dans les mêmes conditions que celles indiquées ci-dessus à l'exemple 17.

3. Analyse de l'ADN

Afin d'étudier la synthèse de l'ADN, les cellules MCF-7*ras* ont été marquées par incubation avec 1 µCi de [$H^3$]-thymidine (activité spécifique 82 Ci/mmol ; Amersham Life Science Inc., UK) ou avec 10 µM de bromodeoxuridine (BrdU, Boehringer France) pendant 6 heures en atmosphère humide contenant 5% de $CO_2$. Les cellules incubées avec la $[H^3]$-thymidine ont été appauvries à l'aide d'un système d'appauvrissement semi-automatique Skatron (Skatron, Lier, Norvège). Après filtration à l'aide d'un filtre en fibres de verre, la radioactivité incorporée a été mesurée. Après fixation des cellules et dénaturation de l'ADN marqué au BrdU, 100 μl/puits de sérum anti-BrdU-POD (Boehringer, France) ont été ajoutés. Les plaques ont été incubées pendant 30 mn. Le complexe BrdU/anti-BrdU-POD a été détecté par une réaction de type ELISA, les produit de réaction étant quantifiés par mesure de l'absorbance à 490 nm à l'aide d'un lecteur de micro plaques (Biorad, France). Les résultats du test ELISA sont corrélés avec les valeurs obtenues au test d'incorporation de la $[H^3]$-thymidine.

4. Milieu expérimental conditionné

Ce milieu est utilisé pour étudier l'action des produits testés sur l'inhibition de l'action des facteurs de croissance cellulaire.

Des cellules MCF-7*ras* ($5.10^6$) ont été cultivées jusqu'à une confluence de 80% dans un milieu DMEM additionné de 10% de SVF, lavées 2 fois au tampon PBS puis incubées en milieu DMEM pendant 24 heures à 37°C. Le milieu ainsi conditionné par les cellules MCF-7*ras* (milieu conditionné) a ensuite été appauvri, centrifugé, puis stocké à -80°C avant emploi.

Des cellules de fibroblastes BALB/c3T3 ($10^5$ cellules) ont été mises en culture dans du DMEM additionné de 10% de SVF dans des plaques de 24 puits (Polylabo, France) et cultivées jusqu'à confluence, lavées deux fois avec du DMEM, et incubées pendant 24 heures en milieu DMEM sans sérum mais contenant 0,1% de BSA (Sigma, St-Louis, MO).

Après ce traitement, les cellules étaient quiescentes. Le milieu a ensuite été remplacé par un mélange de 0,75 ml/puits de milieu conditionné par les cellules MCF-7*ras* et de 0,25 ml/puits de DMEM en présence ou en absence des produits à tester (NaPA et LS4 DMCB) à différentes concentrations. Après 48 heures de traitement, les cellules ont été dénombrées à l'aide d'un compteur de cellules (Coulter).

5. Analyse du cycle cellulaire

Pour analyser le cycle cellulaire, les cellules ont été ensemencées dans des plaques à raison de $5.10^4$ cellules/ ml et par puits en milieu DMEM additionné de 2% de SVF. Après 24 heures de culture, les différents produits à tester ont été ajoutés.

Après 72 heures, les cellules MCF-7ras ont été incubées en présence de BrdU pendant 4 heures. Les cellules ont ensuite été lavées avec du tampon PBS et fixées avec de l'éthanol à 70%. Le BrdU incorporé dans les cellules est révélé avec de l'anti-BrdU conjugué à de l'isothiocyanate de fluorescéine (Boehringer Mannheim). Les cellules sont ensuite centrifugées et remises en suspension dans une solution colorante contenant 50 μl/ml de iodure de propidium (Boehringer Mannheim) pendant 10 mn. Les cellules colorées sont ensuite analysées avec un FACScan (Coulter).

6. Évaluation de l'apoptose

Plusieurs fois au cours du temps, la viabilité des cellules a été déterminée par exclusion au bleu trypan. Les mêmes cellules ont été centrifugées et lavées avec un tampon annexine (Boehringer Mannheim). La translocation de la phosphatidylsérine a été déterminée avec de l'annexine V marquée au FITC et après traitement à l'iodure dé propidium, les cellules ont été analysées à l'aide d'un FACScan (Coulter).

7. Produits testés

Dans cet exemple, les effets des produits suivants, seuls ou en association, à différentes concentrations, ont été étudiés sur la prolifération des cellules MCF-7*ras* après 72 heures d'incubation :

- NaPA vendu par la société Seratec, a été dissous dans de l'eau distillée ;
- Dérivés de dextrane LS4 DMCB, DMCB7 et LS17.

La structure du DMCB7 est décrite dans l'article de R. Baghéri-Yarmand *et al.*, 1994, In Vitro Cell. Dev. Biol. **30A**, 8822-8824.

**II) Résultats**

Les résultats obtenus montrent qu'après 72 heures de traitement, le NaPA utilisé seul inhibe la prolifération des cellules MCF-7*ras* en fonction de la dose utilisée (70% d'inhibition à 30 et 40 mM). Le dextrane LS4 DMCB inhibe la prolifération des cellules MCF-7*ras* avec peu de différence en fonction de la dose utilisée, l'inhibition est maximale (35%) à une dose de 18,5 mM. Le dextrane DMCB7 inhibe la prolifération des cellules MCF-7*ras* en fonction de la dose utilisée avec 60% d'inhibition à une concentration de 7 mM. On remarque que le dextrane LS4 DMCB est plus efficace que le DMCB7 sur la prolifération des cellules tumorales aux doses inférieures à 4 mM.

L'association entre le dextrane LS4 DMCB ou le dextrane DMCB7 et le NaPA a été analysée à partir des courbes effets-doses de chaque produit utilisé seul (voir Tableaux II, III et IV ci-après). Le dextrane DMCB7, à des doses croissantes, a été testé seul ou association avec une dose constante de NaPA de 5 mM (voir Tableau II ci-après). Le dextrane LS4 DMCB, à des doses croissantes, a été testé seul ou association avec des doses croissantes de NaPA (voir Tableau III ci-après).

La même expérience a été réalisée avec le dextrane LS 17, les résultats sont reportés dans le Tableau IV ci-après.

Le pourcentage d'inhibition I a été calculé comme décrit ci-dessus à l'exemple 17.

L'effet de synergie entre les dextranes DMCB7 ou LS4 DMCB ou LS17 et le NaPA a été calculé à l'aide de l'équation isobole suivante (T. Mosmann, J. Immunol. Methods, 1983, **65**, 55-63) :

$$D = A_c/A_e + B_c/B_e$$

dans laquelle $A_c$ et $B_c$ correspondent respectivement à la concentration en dérivé de dextrane et NaPA utilisés en association, et $A_e$ et $B_e$ correspondent à la concentration de chacun de ces deux composés utilisés seuls et induisant le même effet ; D est l'index de combinaison et $D_m$ est l'index de combinaison moyen. Lorsque D ou $D_m$ est inférieur à 1, alors la combinaison des deux produits a un effet synergique. Lorsque D ou $D_m$ = 1 ou est supérieur à 1, alors la combinaison des deux produits a un effet additif ou antagoniste respectivement.

Lorsque la concentration en dérivé de dextrane utilisé seul ($A_e$), permettant d'obtenir un pourcentage d'inhibition proche de celui qui est obtenu lorsque le dérivé de dextrane est utilisé en association avec le NaPA, est très importante et telle qu'elle ne peut être déterminée de façon précise, alors le rapport $A_c/A_e$ tend vers zéro et par conséquent D = $B_c/B_e$. De telles concentrations en dérivés de dextrane ($A_e$) ont été indiquées dans les tableaux II, III et IV ci-après comme étant simplement supérieures à la concentration reportée pour $A_c$.

Chaque expérience a été répétée 3 fois de façon à pouvoir calculer la significativité statistique (P) de l'index de combinaison (D ou $D_m$) comparativement à l'index de combinaison additive des effets (D = 1) par le test *t* de Student.

TABLEAU II

| DMCB7 $A_c$ en mM | NaPA $B_c$ en mM | % Inhibition I | DMCB7 $A_e$ en mM | NaPA $B_e$ en mM | D : Index de Combinaison | $D_m$ : Index de Combinaison moyen |
|---|---|---|---|---|---|---|
| 0,05 | 5 | 10 | 0,4 | 1 | 5,12 | > 2 |
| 0,3 | 5 | 20 | 2,5 | 5 | 1,12 | 1,5 |
| 1 | 5 | 38 | 3,85 | 10,7 | 0,71 [a] | 0,54[a] |
| 1,27 | 5 | 42 | 4,5 | 12 | 0,66 [a] | 0,56 [a] |
| 1,7 | 5 | 45 | 5 | 13 | 0,72 [a] | 0,5 [a] |
| 2 | 5 | 50 | > 7,5 | 20 | 0,25 [a] | 0,58 [a] |
| 5 | 5 | 60 | > 7,5 | 29 | 0,17 [a] | 0,17 [a] |
| 7 | 5 | 70 | > 7,5 | 36 | 0,14 [a] | 0,14 [a] |

[a] : P < 0,05

TABLEAU III

| LS4 $A_c$ en mM | NaPA $B_c$ en mM | % Inhibition I | LS4 $A_c$ en mM | NaPA $B_e$ en mM | D : Index de Combinaison | $D_m$ : Index de Combinaison moyen |
|---|---|---|---|---|---|---|
| 3,7 | 0,75 | 51,4 | > 18,5 | 22 | 0,03 [a] | 0,07 [a] |
| 7,4 | 1,5 | 54,8 | > 18,5 | 23 | 0,065 [a] | 0,11 [a] |
| 14,8 | 3 | 55 | > 18,5 | 23,3 | 0,13 [a] | 0,22 [a] |
| 18,5 | 4 | 60,2 | > 18,5 | 29,3 | 0,14 [a] | 0,26 [a] |

[a] : P < 0,05

TABLEAU IV

| LS17 $A_c$ en mM | NaPA $B_c$ en mM | % Inhibition I | LS17 $A_e$ en mM | NaPA $B_e$ en mM | D : Index de Combinaison | $D_m$ : Index de Combinaison moyen |
|---|---|---|---|---|---|---|
| 1,53 | 0,5 | 0 | 0 | 0 | - | - |
| 3 | 1 | 16 | 7,5 | 0,76 | 0,54 [a] | 0,32 [a] |
| 6 | 2 | 17,3 | > 12 | 0,83 | 2,4 | 1,6 |
| 12 | 4 | 17,3 | > 12 | 0,83 | 4,8 | 8,48 |

[a] : P < 0,05

Les résultats reportés dans le Tableau II montrent une synergie d'action de l'association sur l'inhibition de la prolifération des cellules (70% d'inhibition) pour 5 mM de NaPA et des concentrations en DMCB7 supérieures à 1. Des concentrations en DMCB7 inférieures à 1 mM induisent un effet additif.

Les résultats reportés dans le Tableau III montrent une synergie d'action du NaPA (0,75 mM à 4 mM) associé au LS4 DMCB (de 3,7 à 18,5 mM) sur l'inhibition de la prolifération cellulaire. Cette synergie d'action permet d'obtenir une $IC_{50}$ (concentration minimale induisant 50% d'inhibition) de 4 mM, alors que cette $IC_{50}$ est 5 fois plus élevée lorsque le NaPA est utilisé seul.

Les résultats reportés dans le Tableau IV montrent qu'une synergie d'action entre le LS17 et le NaPA est mise en évidence pour les concentrations respectives de 3 et de 1 mM.

Par conséquent, l'association d'au moins un sel d'un acide phénylalkylcarboxylique de formule (I') et d'au moins un dextrane présente un effet de synergie sur l'inhibition de la prolifération des cellules tumorales.

L'analyse de la répartition des cellules MCF-7ras traitées par le NaPA seul (10 mM), le dextrane LS4 DMCB seul (18,5 mM) et leur association (NaPA : 10mM; LS4 DMCB : 18,5 mM) dans les différentes phases du cycle cellulaire a été réalisée après 48 heures de traitement.

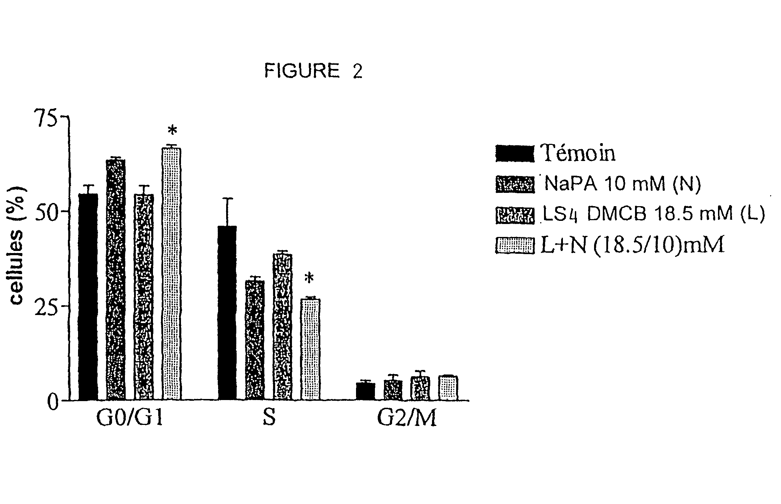

Les résultats sont reportés sur la figure 2.

Ces résultats montrent que le traitement par le NaPA induit un blocage des cellules en phase $G_0/G_1$ ($G_0$ : phase de repos ; $G_1$ : interphase) concomitant à un blocage de la réplication de l'ADN. Lorsque le dextrane LS4 DMCB et le NaPA sont associés, l'inhibition de la réplication de l'ADN et la répartition des ellules en phase $G_0/G_1$ sont augmentés. L'association d'au moins un dextrane et d'au moins un sel d'un acide phénylalkylcarboxylique de formule (I') permet donc le blocage des cellules MCF-7*ras* en phase $G_0/G_1$ et renforce ainsi l'effet cytostatique de chacun des produits utilisés seuls.

En ce qui concerne l'apoptose, il a été montré (Adam *et al*., Cancer Res., 1997, **57**, 1023-1029) que la NaPA induisait une apoptose seul ou en association avec le tamoxifène.

Dans le cadre de la présente invention, il a été observé que l'association de NaPA à 5 mM avec du DMCB7 ou du LS4 DMCB induit une plus forte apoptose des cellules MCF-7*ras* que les produits utilisés séparément.

**EXEMPLE 18 : ÉTUDE DE L'EFFET ANTITUMORAL *IN VIVO* D'UN SEL DE L'ACIDE PHÉNYLALKYLCARBOXYLIQUE DE FORMULE (I') ASSOCIE A UN DEXTRANE DE FORMULE (II) SUR UNE TUMEUR DE SEIN NON-HOR-MONO-DEPENDANTE (CELLULES MCF-7*ras*)**

Du NaPA vendu par la société Seratec, a été dissous dans de l'eau distillée.

Dans cet exemple, le dérivé de dextrane LS4 DMCB, tel que décrit ci-dessus a été utilisé.

50 souris athymiques femelles, âgées de 3 semaines, provenant du laboratoire Harlan (Gannat, France) ont été gardées dans une pièce dont la température était contrôlée et soumise à un cycle lumière / obscurité en alternance toutes les 12 heures.

Les souris ont été approvisionnées en eau et en nourriture.

Des cellules de la lignée tumorale MCF-7*ras* telles que décrites ci-dessus à l'exemple 17 ont été cultivées dans un milieu DMEM additionné de 10% de SFV jusqu'à une confluence de 80%. Après centrifugation, les cellules ont été remises en suspension dans du DMEM additionné de 10% de SVF.

Ces cellules ont ensuite été inoculées à chaque souris par voie sous-cutanée, à proximité de la glande mammaire, à raison de $5.10^6$ cellules par animal.

Après trois semaines d'inoculation, 70% des souris infectées ont présenté une tumeur palpable sous-cutanée.

Le volume des tumeurs (V) a été calculé de la façon suivante :

$V = (4/3)\,\pi\,(r_1)^2\,(r_2)$ ; $r_1$ représentant le plus petit rayon de la tumeur et $r_2$ le plus grand rayon de la tumeur.

Quatre semaine après l'inoculation des cellules tumorales, le volume moyen des tumeurs était de 10 mm$^3$ environ.

Quatre semaines après l'inoculation, des solutions de NaPA (40 mg/kg) ou de dextrane LS4 DMCB (150 mg/kg) ou d'une association de NaPA (40 mg/kg) et de dextrane LS4 DMCB (150 mg/kg), préparées dans 0,1 ml de chlorure de sodium, ont été injectées aux souris, à raison de deux fois par semaine, pendant 7 semaines pour les solutions de NaPA seul et de dextrane LS4 DMCB seul. Les injections de l'association NaPA et de dextrane LS4 DMCB ont été arrêtées au bout de six semaines.

Pour chaque solution testée, 10 souris choisies arbitrairement parmi les 50 souris initialement inoculées, ont été soumises au traitement.

Pendant la même période, un groupe de 10 souris a subi une injection de 0,1 ml de chlorure de sodium pour servir de groupe témoin.

Le volume de chaque tumeur a été mesuré une fois par semaine.

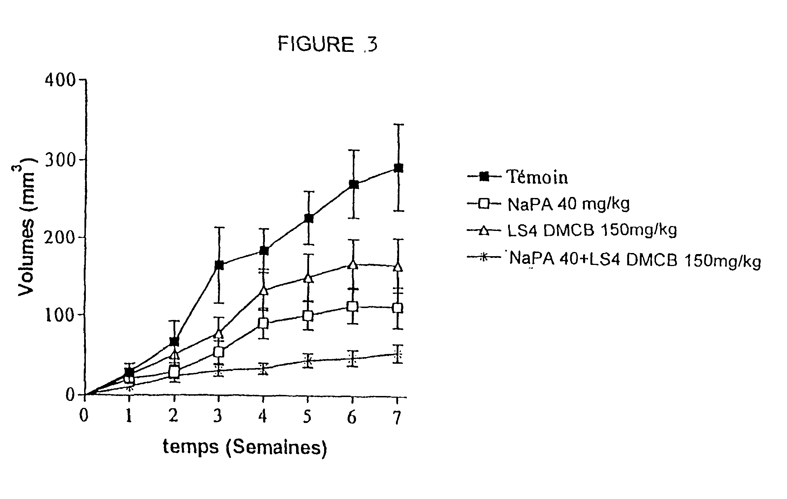

Dans la figure 3, les résultats sont présentés sous forme de moyenne avec un intervalle de confiance de 95%.

Les différentes comparaisons statistiques ont été réalisées à l'aide des tests de ANOVA et de Mann-Whitney dans un modèle linéaire à variables multiples.

Les résultats obtenus apparaissent sur la figure 3 dans laquelle le volume de la tumeur en mm$^3$ est exprimé en fonction du temps en semaines, les carrés pleins correspondent aux résultats obtenus sur le groupe de souris témoins, les carrés ouverts correspondent aux résultats obtenus sur le groupe de souris auquel a été administré le NaPA seul, les losanges correspondent aux résultats obtenus sur le groupe de souris auquel a été administré le dextrane LS4 DMCB seul et les astérisques correspondent aux résultats obtenus sur le groupe de souris auquel a été administré le mélange de NaPA et dextrane LS4.

Après 7 semaines de traitement, on observe que l'injection de NaPA a inhibé la croissance des cellules tumorales de 59% (P=0,049).

Après 4 semaines de traitement, on observe que l'injection de dextrane LS4 DMCB a inhibé la croissance des cellules tumorales de 38% (P=0,0084), cependant la croissance de ces cellules a repris après 7 semaines (P>0,05).

L'injection de la solution contenant l'association de NaPA et de dextrane LS4 DMCB a entraîné une inhibition de la croissance des cellules tumorales de 83% et aucune reprise de la prolifération cellulaire n'a ensuite été observée (P=0,046).

Aucune toxicité apparente de ces produits n'a été observée pendant la durée du traitement.

L'ensemble de ces résultats montre que l'association d'au moins un sel d'un acide phénylalkylcarboxylique de formule (I) et d'au moins un dérivé de dextrane de formule (II) présente une synergie d'action qui permet de réduire de manière très importante la croissance des cellules tumorales, ceci de façon nettement supérieure à la réduction de la croissance des cellules tumorales observée avec chacun des produits utilisés séparément. Ces résultats *in vivo* confirment la synergie d'action sur l'inhibition de la prolifération cellulaire observée *in vitro*.

**EXEMPLE 19 : ÉTUDE DE L'EFFET ANTITUMORAL *IN VIVO* D'UN SEL DE L'ACIDE PHÉNYLALKYLCARBOXYLIQUE DE FORMULE (I) CONJUGUE A UN DEXTRANE DE FORMULE (II) SUR UNE TUMEUR DE SEIN NON-HOR-MONO-DEPENDANTE (CELLULES MCF-7*ras*)**

Dans cet exemple, le composé conjugué de formule (III) synthétisé à l'exemple 1 ci-dessus a été utilisé (LS17-NaPAC).

Deux doses différentes de LS17-NaPAC ont été testées sur des souris athymiques femelles, selon le protocole décrit ci-dessus à l'exemple 18 :

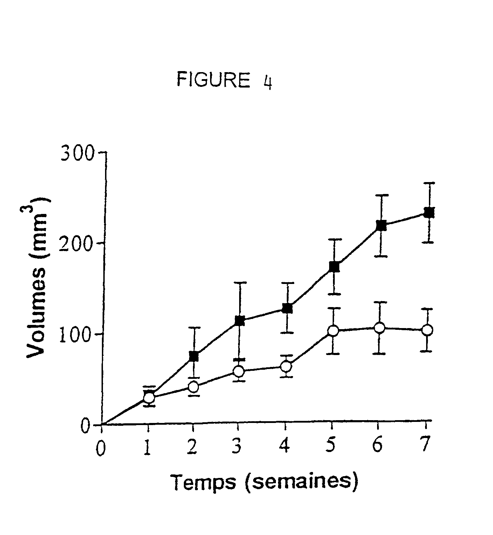

Le traitement des souris par des doses de 15 mg/kg de LS17-NaPAC a été réalisé et aucune toxicité apparente n'a été relevée. Les résultats obtenus sont reportés sur la figure 4, dans laquelle les carrés pleins représentent les résultats obtenus sur le groupe de souris témoin n'ayant reçu aucun traitement et les cercles représentent les résultats obtenus sur le groupe de souris ayant reçu des doses de 15 mg/kg de LS17-NaPAC.

Ces résultats montrent que le LS17-NaPAC à la dose de 15 mg/kg inhibe de 57% la croissance des tumeurs MCF-7*ras* (P = 0,0069) après 6 semaines de traitement, de la même façon que le NaPa utilisé seul à une dose de 40 mg/kg (voir exemple 18 ci-dessus), alors que le dextrane LS17 utilisé seul n'a pas d'effet sur la prolifération des cellules MCF-7*ras in vitro.* Ces résultats montrent que les doses efficaces des composés utilisés sous la forme conjuguée sont plus faibles que celles des composés utilisés sous la forme associée, et que par conséquent, la synergie d'action est encore plus marquée dans le cas des composés conjugués de formule (III).

**EXEMPLE 20 : ÉTUDE COMPARATIVE *IN VIVO* DE L'EFFET ANTITUMORAL DU LS17-NaPAC ET DU NaPA SEUL A CONCENTRATIONS ÉQUIVALENTS SUR UN MELANOME HUMAIN (CELLULES 1205LU)**

Selon le protocole décrit ci-dessus à l'exemple 17, des cellules de mélanome humain 1205LU ont été inoculées par voie sous-cutanée à un groupe de 14 souris athymiques femelles à raison de $10^6$ cellules par animal. Après quatre jours d'inoculation, 100% des souris infectées ont présenté une tumeur palpable sous-cutanée.

Pendant une durée de cinq semaines, et à raison de deux fois par semaine, un premier groupe de 4 souris a subi une injection de 100 µl de chlorure de sodium pour servir de groupe témoin, un deuxième groupe de 4 souris a subi une injection de LS17-NaPAC à une dose de 60 mg/kg/100 µl, et un troisième groupe de 6 souris a subi une injection de NaPA à une dose de 6,9 mg/kg/100 µl.

Il est important de noter qu'une dose de 60 mg/kg/100 µl de LS17-NaPAC contient 6,9 mg/kg/100 µl de NaPA.

Le volume de chaque tumeur a été mesuré avant la première injection et ensuite une fois par semaine pendant les cinq semaines de traitement. Il a été calculé selon l'équation définie ci-dessus à l'exemple 17.

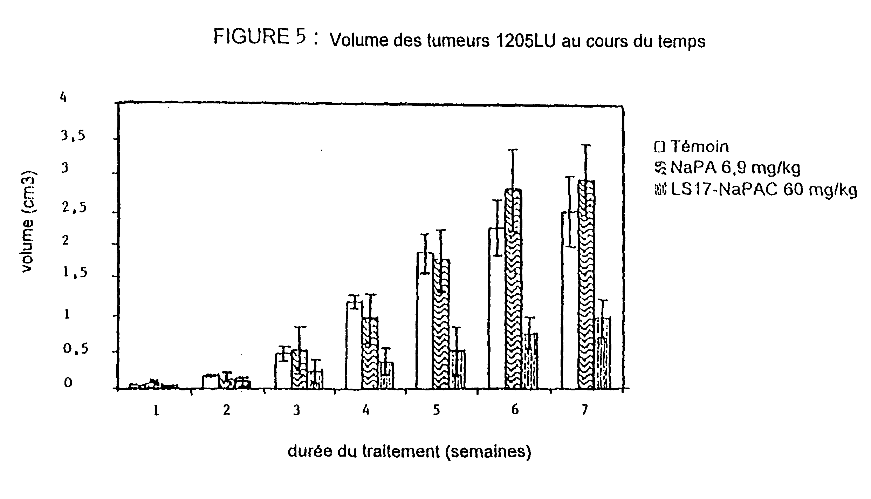

Les résultats ont été soumis à un test non paramétrique et sont reportés sur la figure 5. On peut constater que la NaPA utilisé seul ne semble pas avoir d'effet significatif sur la croissance des tumeurs. Par contre, la LS 17-NaPAC utilisé à une dose de 60 mg/kg inhibe la croissance des tumeurs (p = 0,001) quand on le compare au NaPA.

Par conséquent, la conjugaison du NaPA avec le LS17-DMCB est plus efficace que le NaPA utilisé seul et à une concentration équivalente sur la diminution du volume des tumeurs obtenues à partir des cellules de mélanome 1205LU.

D'autre part, à la fin du traitement, les souris ont été sacrifiées de façon à pouvoir déterminer la masse de chacune des tumeurs. Les résultats obtenus montrent que les courbes exprimant la masse des tumeurs prélevées chez les souris non traitées (groupe témoin) et celle des tumeurs prélevées chez les souris traitées par le NaPA seul à une dose de 6,9 mg/kg ou par le LS 17-NaPAC à une dose de 60 mg/kg confirment les résultats obtenus précédemment. Il n'y a pas de différence significative entre la masse des tumeurs prélevées chez les souris non traitées et celle des tumeurs prélevées chez les souris traitées par le NaPA seul. Le traitement au NaPA seul à une dose de 6,9 mg/kg semble donc non significatif, contrairement à celui au LS17-NaPAC à une dose équivalente de 60 mg/kg (p < 0,005).

**EXEMPLE 21 : ÉTUDE *IN VITRO* DE L'ACTIVITE ANTIANGIOGENIQUE DU LS17-NaPAC, DU LS17-DMCB SEUL ET DU NaPA SEUL A CONCENTRATIONS ÉQUIVALENTES (CELLULES HUVEC)**

Il a été montré précédemment que le NaPA d'une part et un dextrane fonctionnalisé (CMDB7) d'autre part avaient des activités anti-angiogéniques, anti-invasives et anti-métastatiques *in vitro* et *in vivo* (Adam *et al*., Cancer Re., 1997, **57**, 1023-1029 ; Bagheri *et al*., Cancer Res. 1999, **59**, 507-510).

Des cellules endothéliales saines immortalisés (HUVEC) ont été ensemencées à raison de 10 000 cellules par puits et pour 100 µl de milieu DMEM additionné de 2% de SVF dans des micro plaques de 96 puits (COSTAR). Le nombre de cellules a été déterminé après avoir réalisé une gamme étalon.

Après 24 heures de culture, les cellules HUVEC ont été incubées avec les différentes concentrations des produits à tester préparés dans du milieu DMEM à 2% de SVF. L'absorbance au moment de l'ajout des produits a été mesurée et comparée à la gamme étalon, de façon à déterminer le nombre de cellules.

Au bout de 72 heures de culture, le surnageant des cellules est enlevé et lavé 1 fois par du tampon PBS contenant du calcium et du magnésium. 100 µl d'une solution de MTT est alors ajoutée et les cellules sont incubées pendant 4 heures. Après 4 heures d'incubation, le MTT est enlevé et les cristaux formés sont dissous par du DMSO pendant 10 minutes sous agitation. La mesure de l'absorbance se fait à 570 nm.

La concentration de 10 µg/ml de LS17-NaPAC testée est comparée à une concentration en NaPA seul de 1,3216 µg/ml ou à une concentration de 8,7 µg/ml de LS17-DMCB seul.

Ces résultats montrent que le NaPA et le LS17-DMCB testés seuls ne semblent pas présenter d'effet inhibiteur sur la prolifération des cellules HUVEC.

Par contre le LS17-NaPAC inhibe la croissante de ces cellules de façon dose dépendante après 72 heures de traitement. 50% d'inhibition est observé pour concentration de l'ordre de 605 µg/ml.

Par conséquent, le LS17-NaPAC inhibe la prolifération des cellules endothéliales HUVEC. On peut donc en déduire que le LS17-NaPAC peut être utilisé pour inhiber la prolifération des cellules endothéliales afin de diminuer la vascularisation des tumeurs (activité anti-angiogénique).

**Revendications**

**1.** Composition pharmaceutique, **caractérisée par le fait qu'**elle comprend, à titre de principe actif, au moins une composition choisie dans le groupe constitué par :

a) un conjugué d'un dérivé de dextrane de formule (II) suivante :

$$DMC_aB_bSu_c \qquad (II)$$

dans laquelle :

- D représente une chaîne polysaccharidique, de préférence constituée par des enchaînements d'unités glucosidiques,
- MC représente des groupes méthylcarboxylique,
- B représente des groupes N-benzylméthylènecarboxamide,
- Su représente des groupes sulfates (sulfatation des fonctions hydroxyle libres, portées par les unités glucosidiques),
- $\underline{a}$, $\underline{b}$ et $\underline{c}$ représentent le degré de substitution (ds), respectivement des groupements MC, B et Su ; $\underline{a}$ étant égal à 0 ou $\leq$ à 2 ; $\underline{b}$ étant égal à 0 ou $\leq$ à 1 ; $\underline{c}$ étant égal à 0 ou $\leq$ à 1 et ; étant entendu que la somme $\underline{a}$ + $\underline{b}$ + $\underline{c}$ $\leq$ à 3 et d'au moins un dérivé d'un acide phénylalkylcarboxylique de formule (I) suivante :

$$R_2\text{-}(CH_2)_n\text{-}COOR_1 \qquad (I)$$

dans laquelle :

- $R_1$ représente un atome d'hydrogène, un atome d'halogène, un atome d'un métal alcalin monovalent tel que le sodium ou le potassium, ou un groupement $-CO(CH_2)_mR_3$,
- $R_2$ et $R_3$ représentent un radical phényle non substitué,
- n et m, identiques, sont des nombres entiers compris entre 1 et 3 inclusivement ;

ledit conjugué répondant à la formule (IIIa) suivante :

$$(DMC_aB_bSu_c)\text{-}(OOC\text{—}(CH_2)_n\text{-}R_2)_d \qquad (IIIa)$$

dans laquelle n est un nombre entier compris entre 1 et 3 inclusivement, $R_2$ représente un radical phényle non substitué et $\underline{d}$, qui est l'indice de substitution (ds) en dérivé de formule (I), est $\geq$ 0,05 et $\leq$ 1,5 et de préférence > 0,15 ; étant entendu que la somme $\underline{a}$ + $\underline{b}$ + $\underline{c}$ $\leq$ à 3 ;
b) une association d'au moins un dérivé de dextrane présentant une masse moléculaire supérieure ou égale à 5000 Da de formule (II) telle que définie ci-dessus et d'au moins un sel d'un acide phénylalkylcarboxylique de formule (I') suivante :

$$R'_2\text{-}(CH_2)_{n'}\text{-}COOR'_1 \qquad (I')$$

dans laquelle :

- $R'_1$ est un atome d'un métal alcalin monovalent tel que le sodium ou le potassium,
- $R'_2$ représente un radical phényle non substitué,
- n' est un nombre entier compris entre 1 et 3 inclusivement ;

EP 1 289 515 B1

c) et leurs mélanges,

et en présence d'un véhicule pharmaceutiquement acceptable et/ou d'un support physiologiquement acceptable.

2. Composition pharmaceutique selon la revendication 1, **caractérisée par le fait que** les dextranes de formule (II) sont choisis parmi ceux dans lesquels $0,5 \leq \underline{a} \leq 1,5$ ; $0,1 \leq \underline{b} \leq 1$ ; et $0 \leq \underline{c} \leq 0,6$.

3. Composition pharmaceutique selon la revendication 1 ou 2, **caractérisée par le fait que** les sels d'acide phénylalkylcarboxylique de formule (I') sont choisis parmi le phénylacétate de sodium, le phénylacétate de potassium, le phénylpropionate de sodium, le phénylpropionate de potassium, le phénylbutyrate de sodium et le phénylbutyrate de potassium.

4. Composition pharmaceutique selon l'une quelconque des revendications précédentes, **caractérisée par le fait que** le rapport de concentration entre le ou les dérivés de dextrane de formule (II) d'une part et les sels d'un acide phénylalkylcarboxylique de formule (I') d'autre part est compris entre 10:1 et 1:1.

5. Composition pharmaceutique selon l'une quelconque des revendications précédentes, **caractérisée par le fait qu'**elle renferme au moins un composé conjugué de formule (IIIa) choisi parmi ceux dans lesquels a > 0,5 ; b > 0,15 ; c = 0 ou < 0,8 et d < 1,5.

6. Composition selon l'une quelconque des revendications précédentes, **caractérisée par le fait qu'**elle présente les activités biologiques suivantes : antiproliférative, cytostatique, nécrosante, pro-apoptotique, anti-angiogénique, anti-métastatique et inhibitrices des facteurs mitogéniques.

7. Composition selon l'une quelconque des revendications précédentes, **caractérisée par le fait qu'**elle est destinée à être administrée par voie systémique.

8. Composition selon la revendication 7, **caractérisée par le fait qu'**elle est destinée à être administrée à des doses variant de 0,1 mg/kg par jour à 200 mg/kg par jour, à raison de deux fois par semaine.

9. Composés conjugués de formule (IIIa) suivante :

$$(DMC_aB_bSu_c)(OOC\text{-}(CH_2)_nR_2)_d \qquad\qquad (IIIa)$$

dans laquelle D ; MC ; C ; B ; Su ; $R_2$ ; $\underline{a}$, $\underline{b}$, $\underline{c}$ et $\underline{d}$ ont les mêmes significations que celles indiquées à la revendication 1.

10. Composés conjugués selon la revendication 9, **caractérisés par le fait qu'**ils sont choisis parmi ceux dans lesquels a > 0,5 ; b > 0,15 ; c = 0 ou < 0,8 et d < 1,5.


**Claims**

1. Pharmaceutical composition, **characterised by** the fact that it comprises, by way of active principle, at least one composition chosen from the group consisting of:

a) a conjugate of a derivative of dextran of the following formula (II):

$$DMC_aB_bSu_c \qquad\qquad (II)$$

in which:

- D represents a polysaccharide chain, preferably consisting of chain formations of glucoside units,

- MC represents methylcarboxylic groups,

20

- B represents N-benzylmethylene carboxamide groups,

- Su represents sulphate groups (sulphation of the free hydroxyl functions, carried by the glucoside units),

- a, b and c represent the degree of substitution (ds), respectively of the groups MC, B and Su; a being equal to 0 or $\leq 2$; b being equal to 0 or $\leq 1$; c being equal to 0 or $\leq 1$ and; it being understood that the sum $a + b + c \leq 3$, and at least one derivative of a phenylalkylcarboxylic acid of the following formula (I):

$$R_2\text{-}(CH_2)_n\text{-}COOR_1 \qquad\qquad (I)$$

in which:

- $R_1$ represents a hydrogen atom, a halogen atom, an atom of a monovalent alkali metal such as sodium or potassium, or a group $-CO(CH_2)_mR_3$,

- $R_2$ and $R_3$ represent a non-substituted phenyl radical,

- n and m, which are identical, are integer numbers between 1 and 3 inclusive; the said conjugate complying with the following formula (IIIa):

$$(DMC_aB_bSu_c)\text{ - }(OOC-(CH_2)_n\text{-}R_2)_d \qquad\qquad (IIIa)$$

in which n is an integer number between 1 and 3 inclusive, $R_2$ represents a non-substituted phenyl radical and d, which is the substitution index (ds) of derivatives of formula (I), is $\geq 0.05$ and $\leq 1.5$ and preferably $> 0.15$; it being understood that the sum $a + b + c \leq 3$;

b) an association of at least one derivative of dextran having a molecular mass greater than or equal to 5000 Da of formula (II) as defined above and at least one salt of a phenylalkylcarboxylic acid of the following formula (I'):

$$R'_2 \text{ - } (CH_2)_{n'} \text{ - } COOR'_1 \qquad\qquad (I')$$

in which:

- $R'_1$ is an atom of a monovalent alkali metal such as sodium or potassium,

- $R'_2$ represents a non-substituted phenyl radical,

- n' is an integer number between 1 and 3 inclusive;

c) and mixtures thereof,

and in the presence of a pharmaceutically acceptable vehicle and/or a physiologically acceptable medium.

2. Pharmaceutical composition according to Claim 1, **characterised by** the fact that the dextrans of formula (II) are chosen from amongst those in which $0.5 \leq a \leq 1.5$; $0.1 \leq b \leq 1$; and $0 \leq c \leq 0.6$.

3. Pharmaceutical composition according to Claim 1 or 2, **characterised by** the fact that the phenylalkylcarboxylic acid salts of formula (I') are chosen from amongst sodium phenylacetate, potassium phenylacetate, sodium phenylpropionate, potassium phenylpropionate, sodium phenylbutyrate and potassium phenylbutyrate.

4. Pharmaceutical composition according to any one of the preceding claims, **characterised by** the fact that the concentration ratio between the dextran derivative or derivatives of formula (II) on the one hand and the salts of a phenylalkylcarboxylic acid of formula (I') on the other hand is between 10:1 and 1:1.

5. Pharmaceutical composition according to any one of the preceding claims, **characterised by** the fact that it contains at least one conjugate compound of formula (IIIa) chosen from amongst those in which a > 0,5; b > 0.15; c = 0 or < 0.8 and d < 1.5.

6. Composition according to any one of the preceding claims, **characterised by** the fact that it has the following biological activities: antiproliferative, cytostatic, necrotising, pro-apoptotic, anti-angiogenic, antimetastatic and inhibitive of mitogenic factors.

7. Composition according to any one of the preceding claims, **characterised by** the fact that it is intended to be administered systemically.

8. Composition according to claim 7, **characterised by** the fact that it is intended to be administered at doses varying from 0.1 mg/kg per day to 200 mg/kg per day, at the rate of twice per week.

9. Conjugate compounds of the following formula (IIIa):

$$(DMC_aB_bSu_c) . (OOC- (CH_2)_nR_2)_d \qquad (IIIa)$$

in which D; MC; C; B; Su; $R_2$; a, b, c and d have the same meanings as those indicated in Claim 1.

10. Conjugate compounds according to Claim 9, **characterised by** the fact that they are chosen from amongst those in which a > 0.5; b > 0.15; c = 0 or < 0.8 and d < 1.5.

**Patentansprüche**

1. Pharmazeutische Zusammensetzung, **dadurch gekennzeichnet, dass** sie als Wirkstoff mindestens eine Zusammensetzung, welche aus der Gruppe ausgewählt ist, bestehend aus:

   a) einem Konjugat eines Dextranderivats der folgenden Formel (II):

$$DMC_aB_bSu_c \qquad (II)$$

   worin:

   - D eine Polysaccharidkette darstellt, die vorzugsweise durch Kettenbildung glucosidischer Einheiten gebildet wurde;
   - MC Methylcarboxylgruppen darstellt;
   - B N-Benzylmethylencarboxamidgruppen darstellt;
   - Su Sulfatgruppen darstellt (Sulfatierung freier Hydroxylfunktionen, die von glucosidischen Einheiten getragen werden);
   - $\underline{a}$, $\underline{b}$ und $\underline{c}$ den Substitutionsgrad (degré des substitution = ds) der Gruppierungen MC, B und Su darstellen; wobei $\underline{a}$ gleich 0 oder $\leq 2$; $\underline{b}$ gleich 0 oder $\leq 1$; $\underline{c}$ gleich 0 oder $\leq 1$ und wobei die Summe $\underline{a} + \underline{b} + \underline{c} \leq 3$

   und mindestens eines Derivats einer Phenylalkylcarbonsäure der folgenden Formel (I):

$$R_2- (CH_2)_n-COOR_1 \qquad (I)$$

   worin:

   - $R_1$ ein Wasserstoffatom, ein Halogenatom, ein einwertiges Alkalimetallatom wie Natrium oder Kalium oder eine Gruppierung $-CO(CH_2)_mR_3$ darstellt;
   - $R_2$ und $R_3$ einen nichtsubstituierten Phenylrest darstellen;
   - n und m, die gleich sind, ganze Zahlen zwischen 1 und 3, einschließlich, sind;

wobei das Konjugat der folgenden Formel (IIIa) entspricht:

$$(DMC_aB_bSu_c)\text{-}(OOC\text{-}(CH_2)_n\text{-}R_2) \tag{IIIa}$$

in der n eine ganze Zahl zwischen 1 und 3, einschließlich, ist; $R_2$ einen nichtsubstituierten Phenylrest darstellt und d, das der Substitutionsgrad (ds) des Derivats der Formel (I) ist, $\geq 0,05$ und $\leq 1,5$ und vorzugsweise $> 0,15$ ist, wobei die Summe a + b + c $\leq$ 3;

b) einer Assoziation mindestens eines Dextranderivats, das eine Molekularmasse von 5000 Da oder darüber aufweist, mit der Formel (II), wie sie oben definiert ist, und mindestens eines Salzes einer Phenylalkylcarbonsäure der folgenden Formel (I'):

$$R'_2\text{-}(CH_2)_{n'}\text{-}COOR'_1 \tag{I'}$$

worin:

- R'$_1$ ein Atom eines einwertigen Alkalimetall wie Natrium oder Kalium ist,
- R'$_2$ einen nichtsubsituierten Phenylrest darstellt,
- n' eine ganze Zahl zwischen 1 und 3, einschließlich, ist;

c) und ihren Gemischen

in Gegenwart eines pharmazeutisch annehmbaren Vehikels und/oder eines physiologisch annehmbaren Trägers umfasst.

2. Pharmazeutische Zusammensetzung nach Anspruch 1, **dadurch gekennzeichnet, dass** die Dextrane der Formel (II) unter denen ausgewählt sind, in denen $0,5 \leq a \leq 1,5$; $0,1 \leq b \leq 1$; und $0 \leq c \leq 0,6$.

3. Pharmazeutische Zusammensetzung nach Anspruch 1 oder Anspruch 2, **dadurch gekennzeichnet, dass** die Phenylalkylcarbonsäuresalze der Formel (I') unter Natriumphenylacetat, Kaliumphenylacetat, Natriumphenylpropionat, Kaliumphenylproprionat, Natriumphenylbutyrat und Kaliumphenylbutyrat ausgewählt sind.

4. Pharmazeutische Zusammensetzung nach einem der vorangehenden Ansprüche, **dadurch gekennzeichnet, dass** das Konzentrationsverhältnis zwischen dem Dextranderivat oder den Dextranderivaten der Formel (II) einerseits und den Salzen einer Phenylalkylcarbonsäure der Formel (I') andererseits zwischen 10:1 und 1:1 liegt.

5. Pharmazeutische Zusammensetzung nach einem der vorangehenden Ansprüche, **dadurch gekennzeichnet, dass** sie mindestens eine konjugierte Verbindung der Formel (IIIa) umfasst, die unter denen ausgewählt ist, bei denen a > 0,5; b > 0,15; c = 0 oder < 0,8 und d < 1,5.

6. Zusammensetzung nach einem der vorangehenden Ansprüche, **dadurch gekennzeichnet, dass** sie die folgenden biologischen Aktivitäten aufweist: antiproliferativ, cytostatisch, nekrotisierend, proapoptotisch, antiangiogen, antimetastatisch und Inhibitoraktivität gegenüber mitogenen Faktoren.

7. Zusammensetzung nach einem der vorangehenden Ansprüche, **dadurch gekennzeichnet, dass** sie dazu bestimmt ist, auf systemischem Weg verabreicht zu werden.

8. Zusammensetzung nach Anspruch 7, **dadurch gekennzeichnet, dass** sie dazu bestimmt ist, in Dosen, die von 0,1 mg/kg pro Tag bis 200 mg/kg pro Tag variieren, zweimal pro Woche verabreicht zu werden.

9. Konjugierte Verbindungen der folgenden Formel (IIIa):

$$(DMC_aB_bSu_c) \cdot (OOC\text{-}(CH_2)_n\text{-}R_2)_d \tag{IIIa}$$

worin D; MC; C; B; Su; $R_2$; a, b, c und d dieselben Bedeutungen, wie die in Anspruch 1 angegebenen, haben.

10. Konjugierte Verbindungen nach Anspruch 9, **dadurch gekennzeichnet, dass** sie unter denen ausgewählt sind, für die a > 0, 5; b > 0,15; c = 0 oder < 0,8 und d < 1,5.

EP 1 289 515 B1

FIGURE 1 : Effets comparés des produits seuls et des produits associés
sur la prolifération des cellules MCF-7

NaPA seul ▦    ▦ Association    ☐ Dextrane LS4 DMCB seul
du NaPA et
du dextrane LS4 DMCB

FIGURE 2

EP 1 289 515 B1

FIGURE 3

temps (Semaines)

EP 1 289 515 B1

FIGURE 4

FIGURE 5 : Volume des tumeurs 1205LU au cours du temps

EP 1 289 515 B1